# EUROPEAN PATENT APPLICATION

(11) **EP 4 446 333 A1**
(43) Date of publication of application: **16.10.2024**
(21) Application number: 22904726.1
(22) Date of filing: 09.12.2022
(51) Int. Cl.: C07K 5/113, C07K 7/06, C07K 14/47, A61K 8/64, A61Q 17/00, A61K 38/00, A61P 35/00, A61P 3/00, A61P 17/00

(54) **NOVEL PEPTIDE DERIVATIVE DERIVED FROM ADIPONECTIN AND USE THEREOF**

(30) Priority: 10.12.2021 KR 20210176940; 01.12.2022 KR 20220165949
(71) Applicant: Seoul National University Hospital, Seoul 03080 (KR); Daegu-Gyeongbuk Medical Innovation Foundation, Daegu 41061 (KR)
(72) Inventor: CHUNG, Jin Ho, Seoul 04428 (KR); KIM, Eun Ju, Seoul 05217 (KR); SUH, Joong Heon, Seoul 04773 (KR); LEE, Ji Hoon, Seoul 04403 (KR); KWAK, Yoon Na, Daegu 41068 (KR); JUNG, Go Ni, Daegu 42628 (KR); PARK, Sun You, Daegu 41122 (KR); CHOI, Myeong A, Daegu 41061 (KR); MIN, Ju Sik, Daegu (KR); LEE, Ha Yeon, Daegu 41069 (KR)
(74) Representative: Gevers Patents
(86) International application number: PCT/KR2022/020076
(87) International publication number: WO 2023/106897

(57) **Abstract**

The present disclosure relates to a novel peptide derivative, etc., the peptide derivative being a partially modified adiponectin receptor peptide which, compared to conventional adiponectin receptor peptides, has higher stability, superior p-AMPK activity, and improved physical properties and activity and thus offers advantages when formulated into a drug. Therefore, the peptide derivative of the present invention can be used to prevent or treat inflammatory skin diseases, wounds, hair loss, fibrosis, metabolic diseases, or cancer, to prevent or alleviate aging, sensitive skin, or wrinkles, or to enhance moisturization.

## Description

### TECHNICAL FIELD

The present disclosure relates to novel peptide derivatives derived from adiponectin and a use thereof.

### BACKGROUND ART

Skin aging is often caused by external and internal processes associated with an increase in skin wrinkles, drooping, and sagging, and external aging is commonly referred to as 'photoaging' because it is mainly caused by repeated exposure to ultraviolet rays (UV). Naturally aged skin is smooth, pale, and has fine wrinkles, but photoaged skin develops thick skin wrinkles and causes pigmentation and telangiectasia.

Subcutaneous fat plays an important role in maintaining energy homeostasis by secreting hormones and adipokine that regulate the metabolism of other tissues. Recently, the fat content of human subcutaneous fat tissue has decreased due to UV, which is an environmental factor that causes various diseases such as photoaging, inflammation, immunosuppression, and cancer, and it has been known that adiponectin, a fat-derived product, has decreased in aged skin, and induces an MMP-1 increase and a decrease in collagen.

There are about 1.3 million or more hairs in the human body, and 100,000 to 150,000 hairs on the head, and the respective hairs have different cycles, and grow, are kept, and lost through three stages of the anagen (the active growth phase), the catagen (the apoptotic regression phase), and the telogen (the resting phase). These cycles are repeated over 3 to 6 years, and as a result, average 50 to 100 hairs per day are normally lost. In general, 'hair loss' means a condition with less or no hairs or furs than normal due to any reason.

Today, the causes of hair loss include internal factors such as the action of male hormones, and external factors such as mental stress in daily life and accumulation of lipid peroxidation in the scalp, and these factors are known to be involved in a complex manner to show hair loss symptoms. In recent years, not only male-pattern hair loss, but also female hair loss population are increasing due to an increase in stress caused by changes in dietary life and social environment, and the population suffering from such abnormal symptoms of scalp and hairs is gradually increasing, and the age thereof is also getting lower.

Hair growth agents currently on the market have problems with lack of clear effects and side effects, and problems of requiring continuous use, and causing side effects such as decreased sexual function, allergies, and depression.

Metabolic diseases refer to syndromes in which risk factors such as obesity, diabetes, high blood pressure, arteriosclerosis, and nonalcoholic fatty liver disease (NAFLD) occur together due to excessive accumulation of nutrients in the body and lack of exercise. Recently, the metabolic diseases were officially named metabolic syndromes or insulin resistance syndromes through the Adult Treatment Program III established by the World Health Organization and the National Heart, Lung, and Blood Institute of the National Institutes of Health in USA. In addition, according to the ATP of the U.S. National Cholesterol Education Program (NCEP) published in 2001, when one patient has three or more of five risk factors of abdominal obesity with a waist circumference of 40 inches (102 cm) or more for men and 35 inches (88 cm) or more for women, triglycerides of 150 mg/dL or more, HDL cholesterol of 40 mg/dL or less for men and 50 mg/dL or less for women, blood pressure of 130/85 mmHg or more, and fasting glucose of 110 mg/dL or more, it is judged to be a metabolic disease. In the case of Asians, when the waist circumference is over 90 for men and 80 for women, it is somewhat adjusted to abdominal obesity, and if such a regulation is applied, there is also a recent research report that in Koreans, about 25% of the total population shows symptoms of metabolic syndrome.

On the other hand, adiponectin is a type of adipokine, which is a protein hormone specifically secreted by adipocytes, and known to play an important role to adjust cardiovascular diseases such as hyperglycemia, hyperinsulinism, obesity, and arteriosclerosis by enhancing the function of insulin and suppressing insulin resistance to block inflammation and prevent fat accumulation in blood vessels. In addition, the adiponectin has a function of suppressing the metastasis and inflammatory response of cancer cells, and promotes not only the proliferation of keratinocytes but also the expression of filaggrin, hyaluronic acid, and extracellular matrix in the skin, thereby performing functions of wound treatment, fibrosis suppression, alleviation of skin wrinkles, moisturization, and the like.

The adiponectin consists of 244 amino acids and consists of a signal sequence, a collagen-like domain located at an N-terminal and a C1q-like globular domain located at a C-terminal. A hexamer and a high molecular complex (HMW complex) of 400 kDa are main oligomers, and the HMW complex is known to be more active than a low molecular complex (LMW complex).

The development of adiponectin-modified peptides, which are known to have various physiological activities in the related art, has been targeted by many domestic and foreign researchers and pharmaceutical companies, but the possibility of final success was relatively low due to difficulties in forming polymers in the body.

Under this background, the present inventors developed short peptide derivatives that were able to be applied to the skin by improving the physical properties and activity of conventional adiponectin-derived peptides, which were difficult to be formulated due to poor solubility, and confirmed the effects on increasing adiponectin expression, promoting hair growth, and suppressing neutral fat, and then completed the present disclosure.

### DISCLOSURE OF THE INVENTION

### TECHNICAL GOALS

An aspect of the present disclosure is to provide a novel peptide derivative.

Another aspect of the present disclosure is to provide a composition for increasing the expression of adiponectin including the peptide derivative as an active ingredient.

Yet another aspect of the present disclosure is to provide a pharmaceutical composition for preventing or treating inflammatory skin diseases, wounds, hair loss, fibrosis, metabolic diseases, or cancer, including the peptide derivative as an active ingredient.

Yet another aspect of the present disclosure is to provide a cosmetic composition for preventing or alleviating inflammatory skin diseases, wounds, hair loss, fibrosis, metabolic diseases, or cancer, including the peptide derivative as an active ingredient.

Yet another aspect of the present disclosure is to provide a pharmaceutical composition for preventing or alleviating aging, sensitive skin, wrinkles or moisturization, including the peptide derivative as an active ingredient.

Yet another aspect of the present disclosure is to provide a cosmetic composition for preventing or alleviating aging, sensitive skin, wrinkles or moisturization, including the peptide derivative as an active ingredient.

However, technical goals to be achieved are not limited to those described above, and other goals not mentioned above are clearly understood by one of ordinary skill in the art from the following description.

### TECHNICAL SOLUTIONS

In order to solve the aspect, the present disclosure provides a peptide derivative represented by [Chemical Formula 1] below.

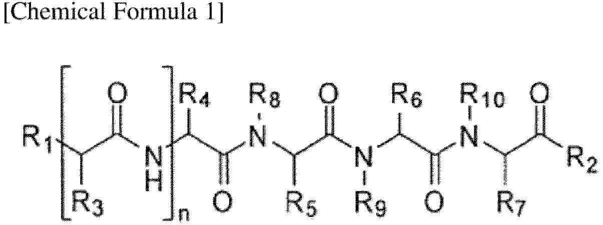

In [Chemical Formula 1],
R₁ is at least one selected from the group consisting of an amino group (NH₂), an acetylamino group (Ac-NH), NH₂-(CH₂CH₂O)ₘ-CH₂CONH, a C₁₅-C₂₀ amide group, and combinations thereof, in which m may be an integer of 1 to 5, desirably 3, and the amide group may be desirably a C₁₆ amide group, that is, a palmitamide group,
R₂ is an amino group or a hydroxyl group (OH),
R₃ is hydrogen (H),
R₄ is at least one selected from the group consisting of a C₁-C₆ acyclic alkyl group, a C₂-C₆ alkylcarboxyl group, a C₁-C₆ alkylamide group, an imidazoylmethyl group, and combinations thereof,
R₅ and R₆ are the same as or different from each other, and are each independently a benzyl group, a phenylethyl group, or an indolylmethyl group, in which the benzyl group is unsubstituted or at least one selected from the group consisting of a hydroxyl group, a trifluoromethyl group (CF₃), a halogen group, a cyano group, a nitro group, a C₁-C₆ acyclic alkyl group, a C₁-C₆ alkoxy group, and combinations thereof,
R₇ is a benzyl group or a C₁-C₆ alkylamino group, wherein the benzyl group is unsubstituted or at least one selected from the group consisting of a hydroxyl group, a halogen group, a cyano group, a nitro group, a C₁-C₆ acyclic alkyl group, a C₁-C₆ alkoxy group, and combinations thereof,
R₃ and R₇ are linked to each other to form a C₁₅-C₂₀ heterocycloalkene group,
R₈, R₉, and R₁₀ are the same as or different from each other, and are each independently hydrogen or a C₁-C₆ alkyl group,
n may be 0 or 1,
when n is 0, R₄ may be a C₂-C₆ alkylcarboxyl group, and R₅, R₆, and R₇ may be benzyl groups,
however, R₁ is NH₂, R₂ is OH, R₃ is hydrogen, R₄ is an isobutyl group, R₅ and R₆ are both hydroxybenzyl groups, R₇ is an unsubstituted benzyl group, and R₈, R₉, and R₁₀ are all hydrogen, except for the case where n is 1. That is, NH₂-GLYYF-OH known as an APN5 peptide is excluded.

In an embodiment of the present disclosure, the R₄ may be at least one selected from the group consisting of 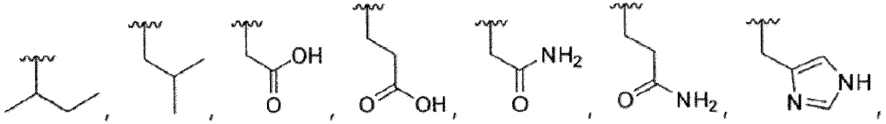 and combinations thereof, but is not limited thereto.

In an embodiment of the present disclosure, the R₅ and R₆ may be the same as or different from each other and each independently at least one selected from the group consisting of 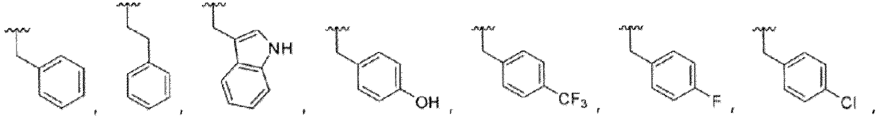 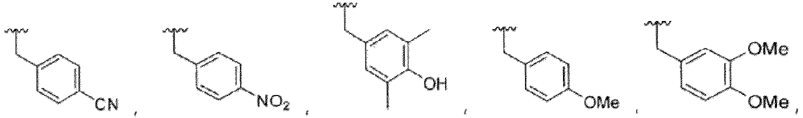 and combinations thereof, but is not limited thereto.

In another embodiment of the present disclosure, the C₁₅-C₂₀ heterocycloalkene group may be at least one selected from the group consisting of 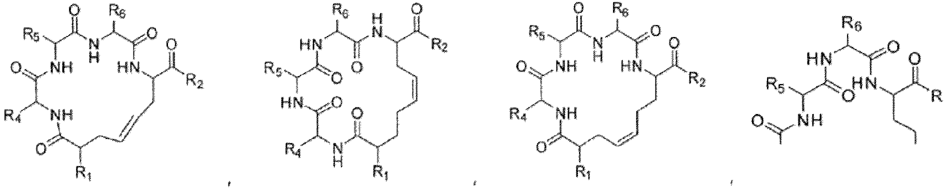 , and combinations thereof, but is not limited thereto.

As another embodiment of the present disclosure, the peptide derivative may be at least one selected from the group consisting of peptide derivatives represented by [Chemical Formula 1-1] to [Chemical Formula 1-56] below, but is not limited thereto (Table 1).

**[Table 1]**

| **Compound No.** | **Chemical Formula No.** | **Chemical Formula** |
|---|---|---|
| DS-1 | 1-1 | 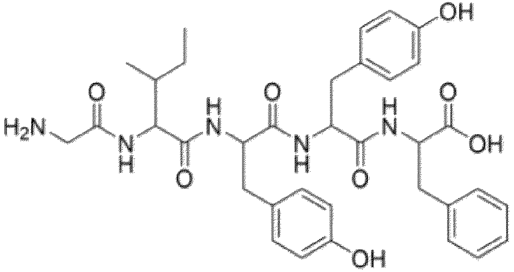 |
| DS-2 | 1-2 | 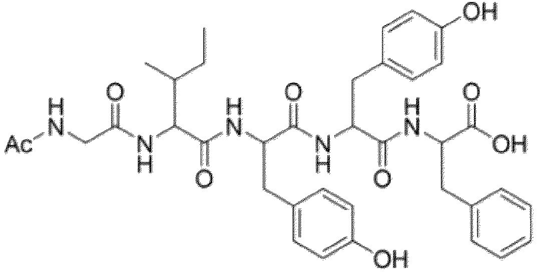 |
| DS-3 | 1-3 | 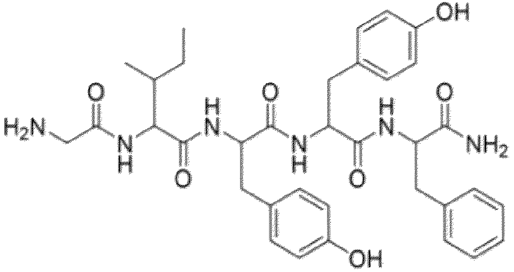 |
| DS-4 | 1-4 | 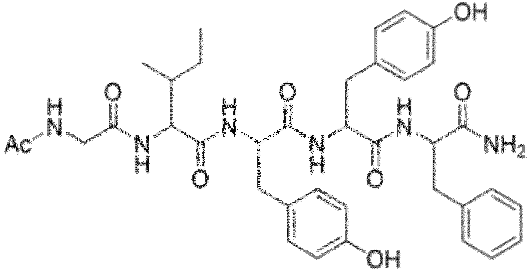 |
| DS-5 | 1-5 | 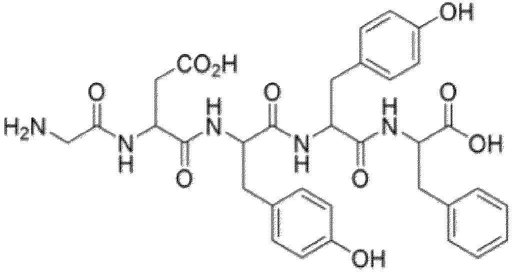 |
| DS-6 | 1-6 | 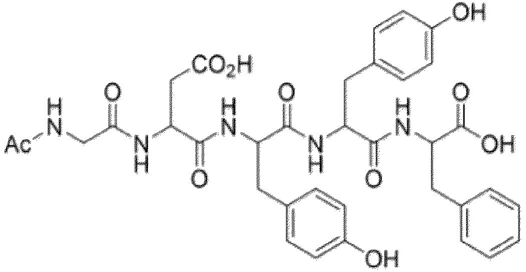 |
| DS-7 | 1-7 | 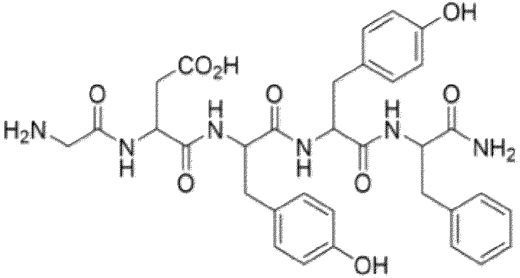 |
| DS-8 | 1-8 | 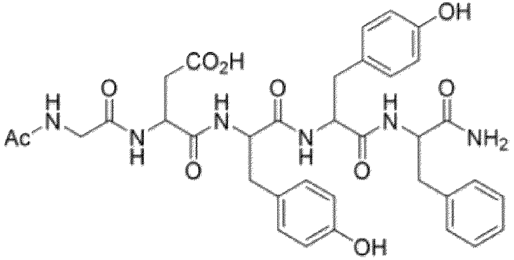 |
| DS-9 | 1-9 | 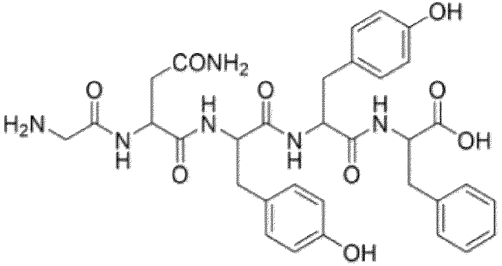 |
| DS-10 | 1-10 | 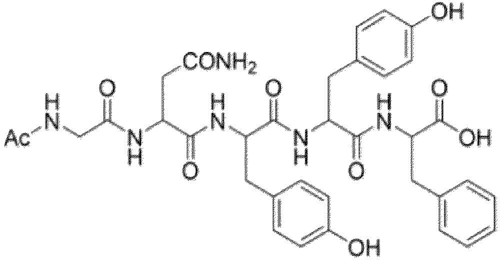 |
| DS-11 | 1-11 | 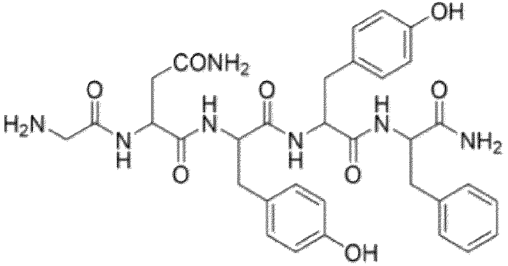 |
| DS-12 | 1-12 | 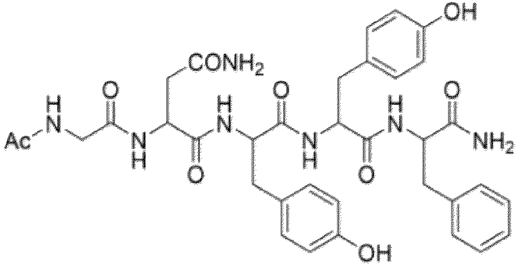 |
| DS-17 | 1-13 | 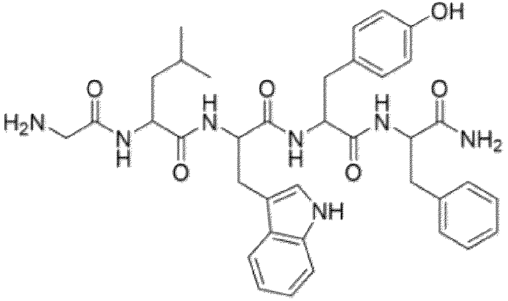 |
| DS-18 | 1-14 | 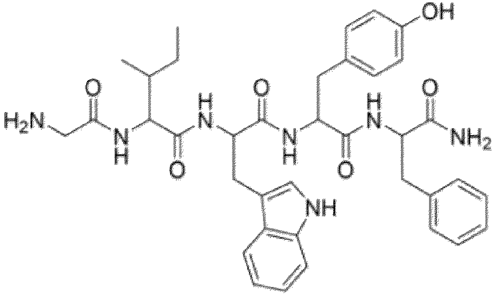 |
| DS-19 | 1-15 | 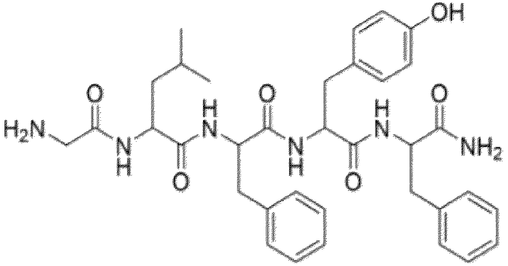 |
| DS-20 | 1-16 | 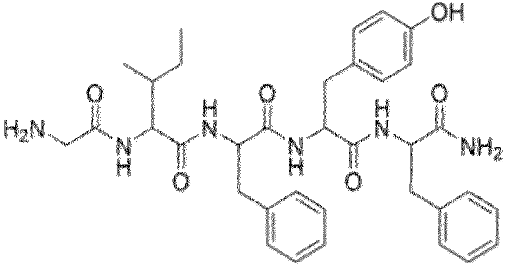 |
| DS-27 | 1-17 | 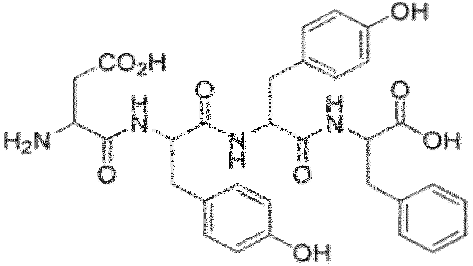 |
| DS-28 | 1-18 | 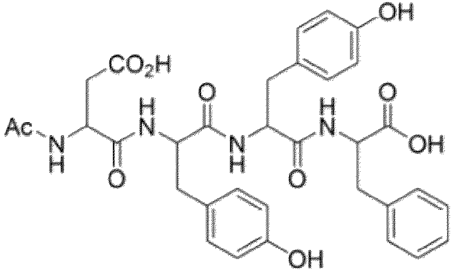 |
| DS-29 | 1-19 | 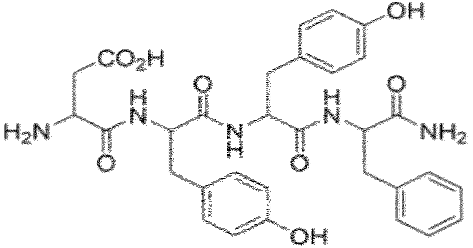 |
| DS-30 | 1-20 | 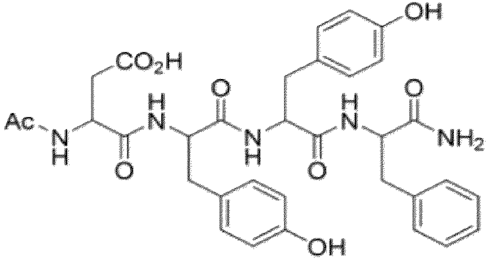 |
| DS-35 | 1-21 | 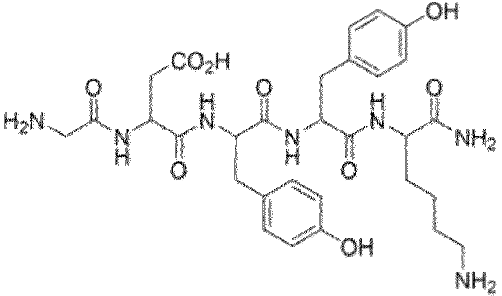 |
| DS-36 | 1-22 | 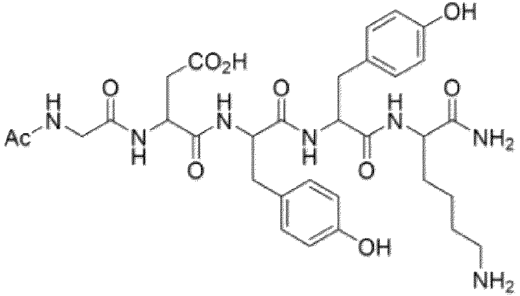 |
| DS-37 | 1-23 | 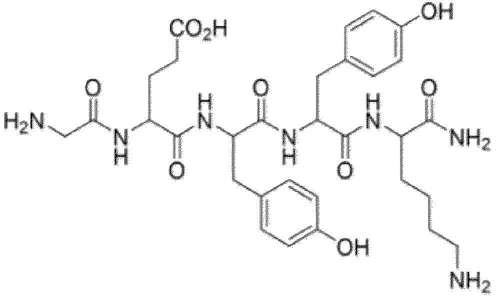 |
| DS-38 | 1-24 | 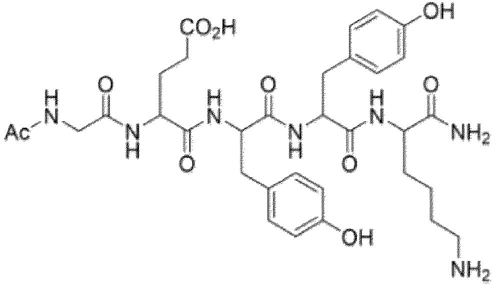 |
| DS-39 | 1-25 | 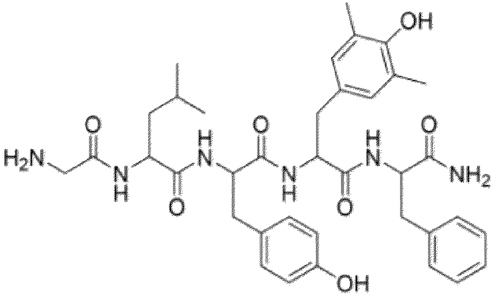 |
| DS-40 | 1-26 | 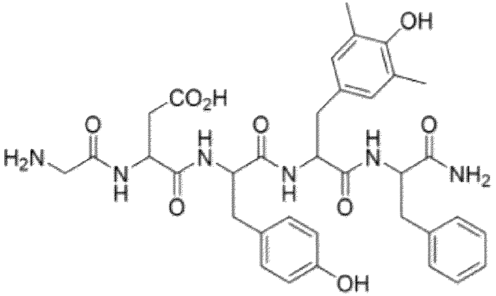 |
| DS-41 | 1-27 | 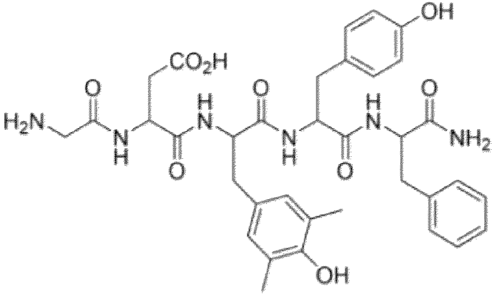 |
| DS-42 | 1-28 | 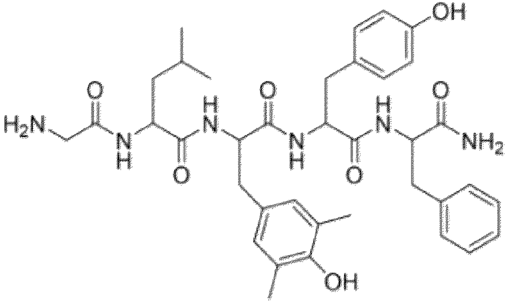 |
| DS-44 | 1-29 | 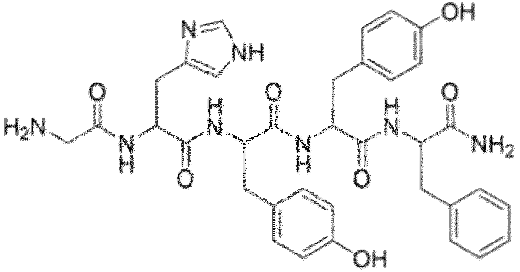 |
| DS-45 | 1-30 | 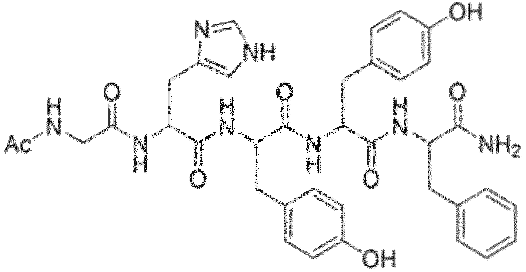 |
| DS-48 | 1-31 | 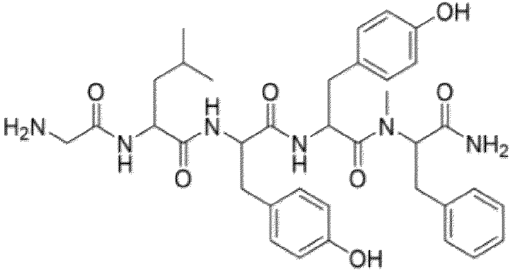 |
| DS-49 | 1-32 | 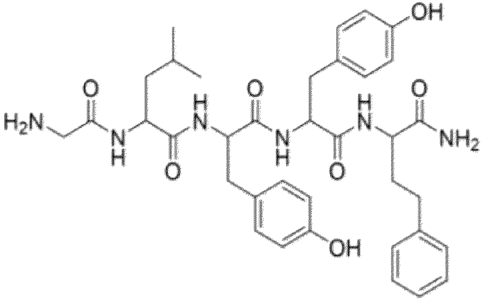 |
| DS-50 | 1-33 | 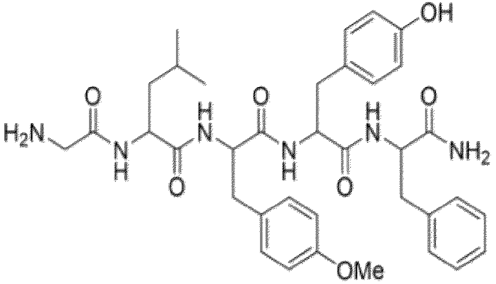 |
| DS-51 | 1-34 | 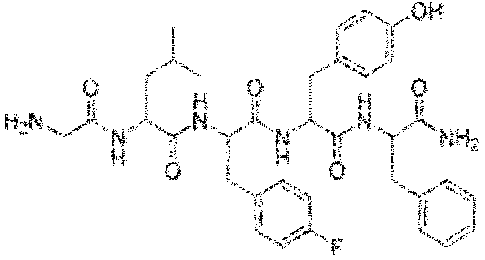 |
| DS-52 | 1-35 | 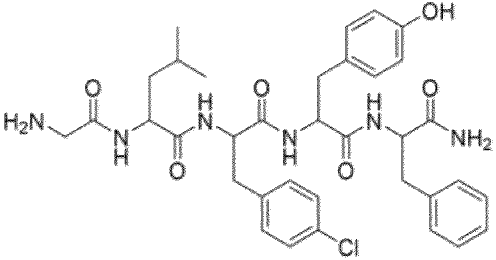 |
| DS-53 | 1-36 | 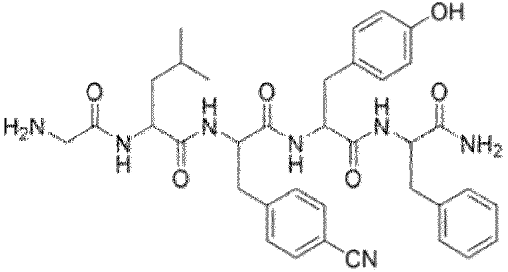 |
| DS-54 | 1-37 | 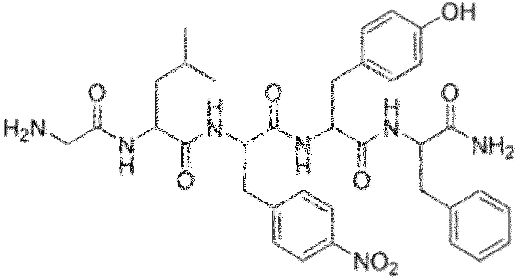 |
| DS-55 | 1-38 | 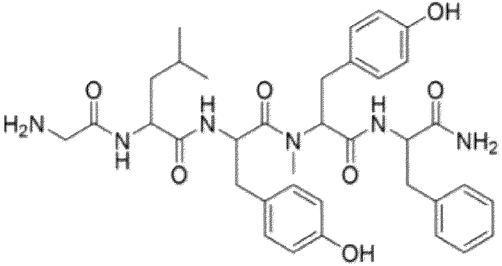 |
| DS-56 | 1-39 | 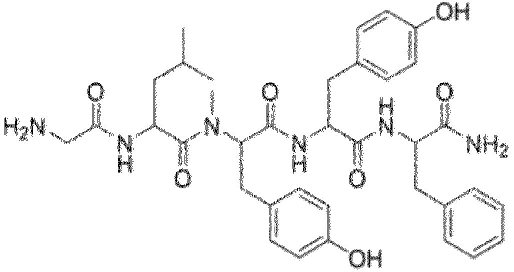 |
| DS-57 | 1-40 | 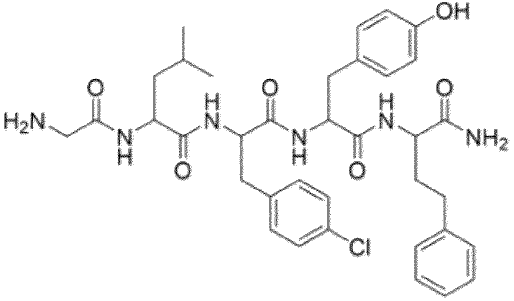 |
| DS-58 | 1-41 | 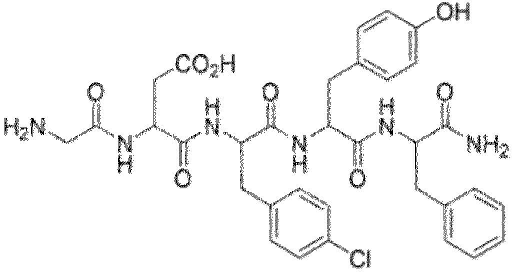 |
| DS-59 | 1-42 | 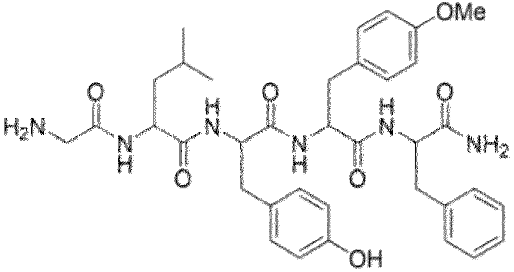 |
| DS-60 | 1-43 | 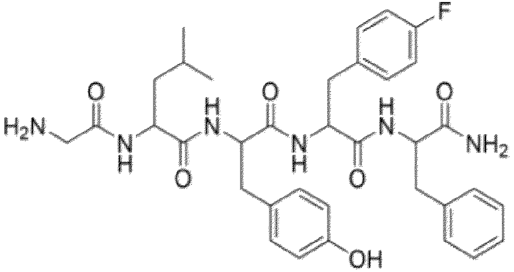 |
| DS-61 | 1-44 | 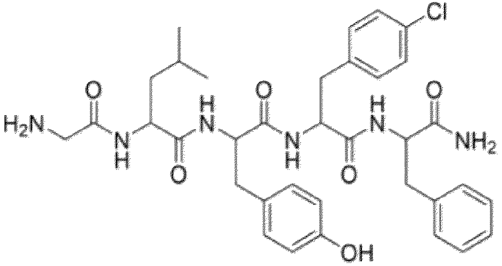 |
| DS-62 | 1-45 | 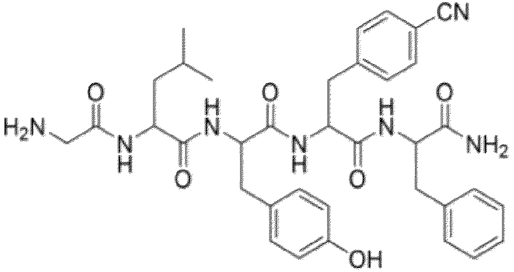 |
| DS-63 | 1-46 | 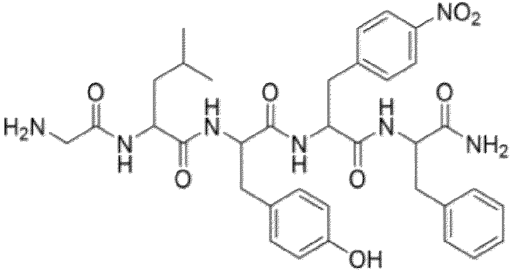 |
| DS-68 | 1-47 | 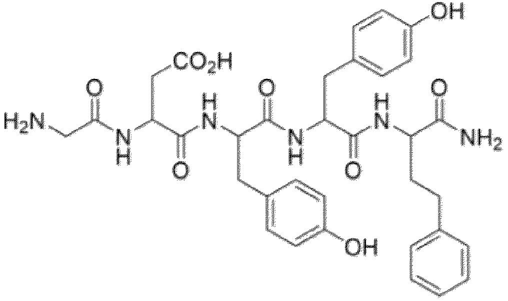 |
| DS-70 | 1-48 | 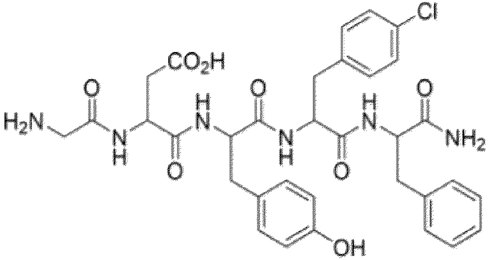 |
| DS-72 | 1-49 | 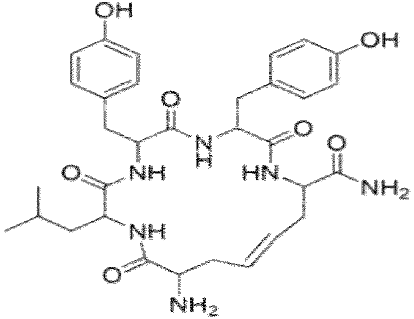 |
| DS-73 | 1-50 | 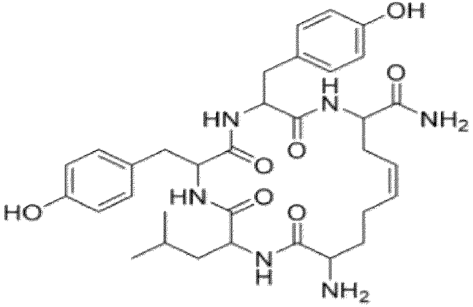 |
| DS-74 | 1-51 | 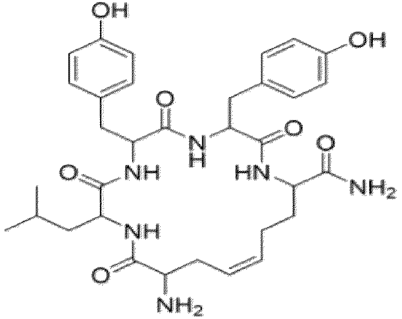 |
| DS-75 | 1-52 | 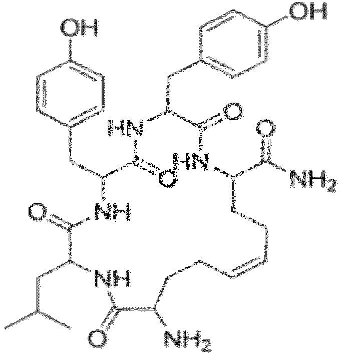 |
| DS-76 | 1-53 | 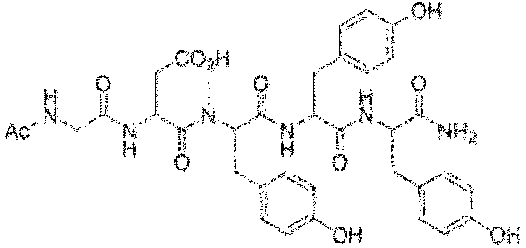 |
| DS-77 | 1-54 | 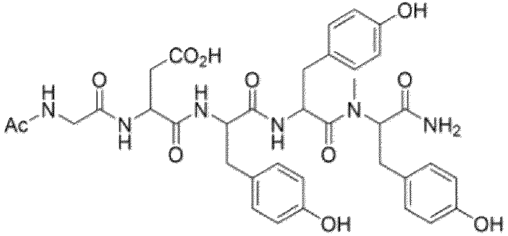 |
| DS-78 | 1-55 | 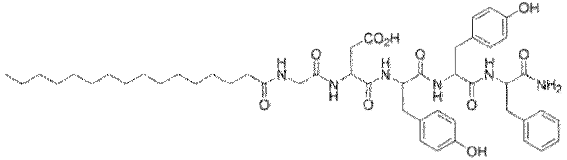 |
| DS-79 | 1-56 | 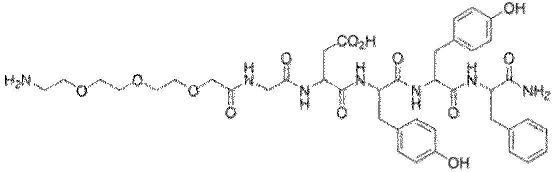 |

In another embodiment of the present disclosure, the peptide derivative may be at least one modified peptide sequence selected from the group consisting of SEQ ID NOs: 1 to 16, but is not limited thereto (Table 2).

**[Table 2]**

| **SEQ ID NO:** | **Peptide sequence** | **Compound No.** | **Chemical Formula No.** |
|---|---|---|---|
| 1 | DYYF | DS-27 | 1-17 |
| | | DS-28 | 1-18 |
| | | DS-29 | 1-19 |
| | | DS-30 | 1-20 |
| 2 | GDFYF | DS-58 | 1-41 |
| 3 | GDYFF | DS-70 | 1-48 |
| 4 | GDYYF | DS-5 | 1-5 |
| | | DS-6 | 1-6 |
| | | DS-7 | 1-7 |
| | | DS-8 | 1-8 |
| | | DS-40 | 1-26 |
| | | DS-41 | 1-27 |
| | | DS-68 | 1-47 |
| | | DS-78 | 1-55 |
| | | DS-79 | 1-56 |
| 5 | GDYYK | DS-35 | 1-21 |
| | | DS-36 | 1-22 |
| 6 | GDYYY | DS-76 | 1-53 |
| | | DS-77 | 1-54 |
| 7 | GEYYK | DS-37 | 1-23 |
| | | DS-38 | 1-24 |
| 8 | GHYYF | DS-44 | 1-29 |
| | | DS-45 | 1-30 |
| 9 | GIFYF | DS-20 | 1-16 |
| 10 | GIWYF | DS-18 | 1-14 |
| 11 | GIYYF | DS-1 | 1-1 |
| | | DS-2 | 1-2 |
| | | DS-3 | 1-3 |
| | | DS-4 | 1-4 |
| 12 | GLFYF | DS-19 | 1-15 |
| | | DS-50 | 1-33 |
| | | DS-51 | 1-34 |
| | | DS-52 | 1-35 |
| | | DS-53 | 1-36 |
| | | DS-54 | 1-37 |
| | | DS-57 | 1-40 |
| 13 | GLWYF | DS-17 | 1-13 |
| 14 | GLYFF | DS-59 | 1-42 |
| | | DS-60 | 1-43 |
| | | DS-61 | 1-44 |
| | | DS-62 | 1-45 |
| | | DS-63 | 1-46 |
| 15 | GLYYF | DS-39 | 1-25 |
| | | DS-42 | 1-28 |
| | | DS-48 | 1-31 |
| | | DS-49 | 1-32 |
| | | DS-55 | 1-38 |
| | | DS-56 | 1-39 |
| 16 | GNYYF | DS-9 | 1-9 |
| | | DS-10 | 1-10 |
| | | DS-11 | 1-11 |
| | | DS-12 | 1-12 |

Further, the present disclosure provides a composition for increasing the expression of adiponectin including the peptide derivative as an active ingredient.

Further, the present disclosure provides a pharmaceutical composition for preventing or treating at least one selected from the group consisting of inflammatory skin diseases, wounds, hair loss, fibrosis, metabolic diseases, and cancer, including the peptide derivative as an active ingredient.

Further, the present disclosure provides a method for preventing or treating at least one selected from the group consisting of inflammatory skin diseases, wounds, hair loss, fibrosis, metabolic diseases, and cancer, including administering the peptide derivative to a subject.

Further, the present disclosure provides a use of the peptide derivative for preparing an agent for preventing or treating at least one selected from the group consisting of inflammatory skin diseases, wounds, hair loss, fibrosis, metabolic diseases, and cancer.

Further, the present disclosure provides a cosmetic composition for preventing or alleviating at least one selected from the group consisting of inflammatory skin diseases, wounds, hair loss, fibrosis, metabolic diseases, and cancer, including the peptide derivative as an active ingredient.

Further, the present disclosure provides a food composition for preventing or alleviating at least one selected from the group consisting of inflammatory skin diseases, wounds, hair loss, fibrosis, metabolic diseases, and cancer, including the peptide derivative as an active ingredient.

Further, the present disclosure provides a pharmaceutical composition for preventing or alleviating at least one selected from the group consisting of aging, sensitive skin, wrinkles and moisturization, including the peptide derivative as an active ingredient.

Further, the present disclosure provides a method for preventing or alleviating at least one selected from the group consisting of aging, sensitive skin, wrinkles and moisturization, including administering the peptide derivative to a subject.

Further, the present disclosure provides a use of the peptide derivative for preparing an agent for preventing or alleviating at least one selected from the group consisting of aging, sensitive skin, wrinkles and moisturization.

Further, the present disclosure provides a cosmetic composition for preventing or alleviating at least one selected from the group consisting of aging, sensitive skin, wrinkles and moisturization, including the peptide derivative as an active ingredient.

Further, the present disclosure provides a food composition for preventing or alleviating at least one selected from the group consisting of aging, sensitive skin, wrinkles and moisturization, including the peptide derivative as an active ingredient.

### EFFECTS OF THE INVENTION

According to an example of the present disclosure, the peptide derivative is a partially modified adiponectin receptor peptide and has higher stability, superior p-AMPK activity, and improved physical properties and activity compared to conventional adiponectin receptor peptides, and thus has advantages when formulated into a drug.

Therefore, the peptide derivative of the present disclosure may be used to prevent or treat inflammatory skin diseases, wounds, hair loss, fibrosis, metabolic diseases, or cancer, and to prevent or alleviate aging, sensitive skin, wrinkles, or moisturization.

Effects of the peptide derivative according to Example of the present disclosure are not limited to those mentioned above, and other effects not mentioned will be clearly understood by those skilled in the art from the following description.

### BRIEF DESCRIPTION OF DRAWINGS

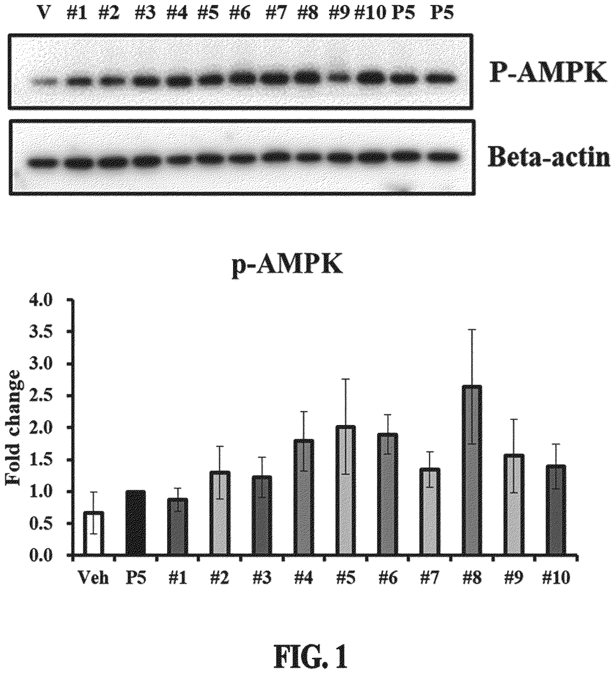

FIG. 1 is a graph showing effects of peptide derivative DS-1 and DS-10 according to the present disclosure represented by [Chemical Formula 1-1] to [Chemical Formula 1-10] on AMPK phosphorylation activity in mouse adipocytes. At this time, V is a vehicle treated with DMSO, and P5 is an APN5 peptide as a positive control and has a sequence NH₂-GLYYF-OH.

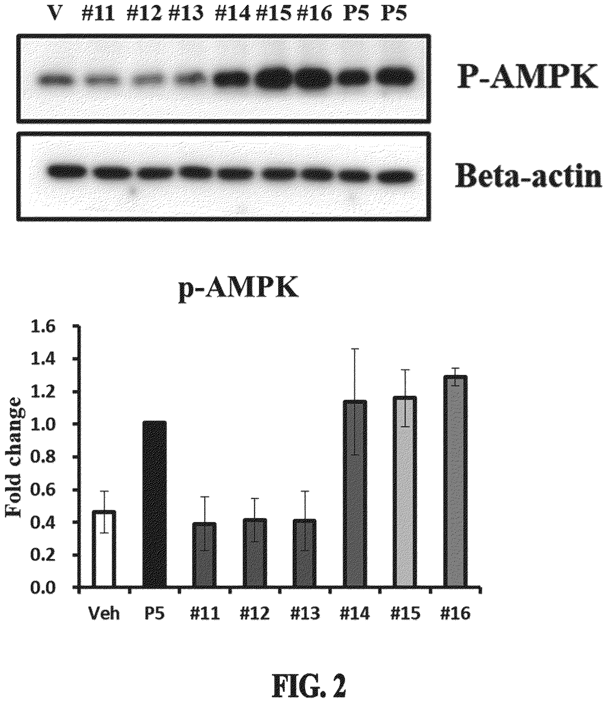

FIG. 2 is a graph showing effects of peptide derivatives DS-11 and DS-12 of the present disclosure represented by [Chemical Formula 1-11] and [Chemical Formula 1-12] on AMPK phosphorylation activity in mouse adipocytes. At this time, V is a vehicle treated with DMSO, and P5 is an APN5 peptide as a positive control and has a sequence NH₂-GLYYF-OH.

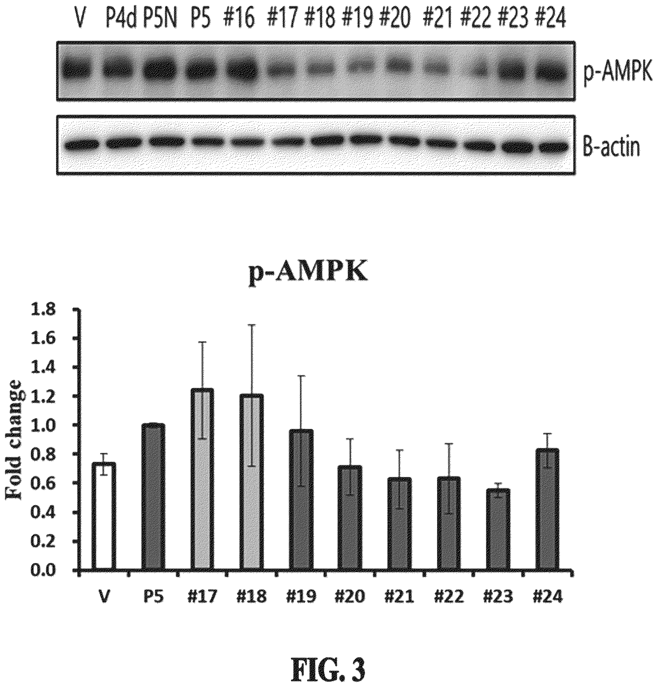

FIG. 3 is a graph showing effects of peptide derivatives DS-17 to DS-20 of the present disclosure represented by [Chemical Formula 1-13] to [Chemical Formula 1-16] on AMPK phosphorylation activity in mouse adipocytes. At this time, V is a vehicle treated with DMSO, and P5 is an APN5 peptide as a positive control and has a sequence NH₂-GLYYF-OH.

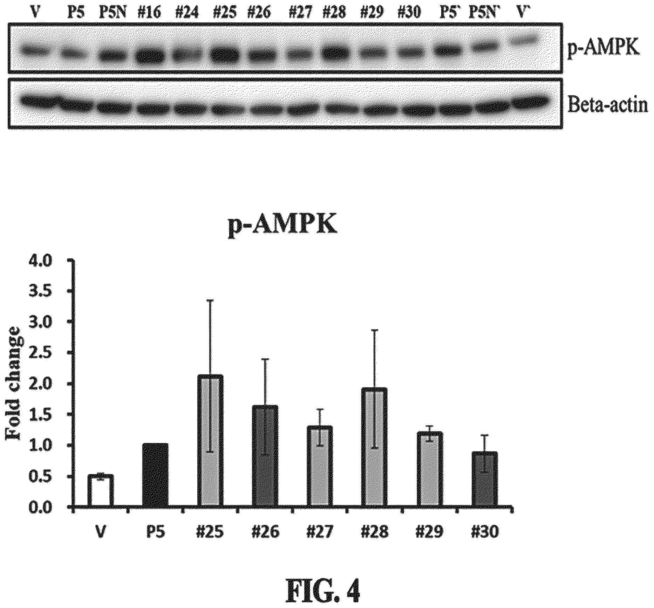

FIG. 4 is a graph showing effects of peptide derivatives DS-27 to DS-30 of the present disclosure represented by [Chemical Formula 1-17] to [Chemical Formula 1-20] on AMPK phosphorylation activity in mouse adipocytes. At this time, V is a vehicle treated with DMSO, and P5 is an APN5 peptide as a positive control and has a sequence NH₂-GLYYF-OH.

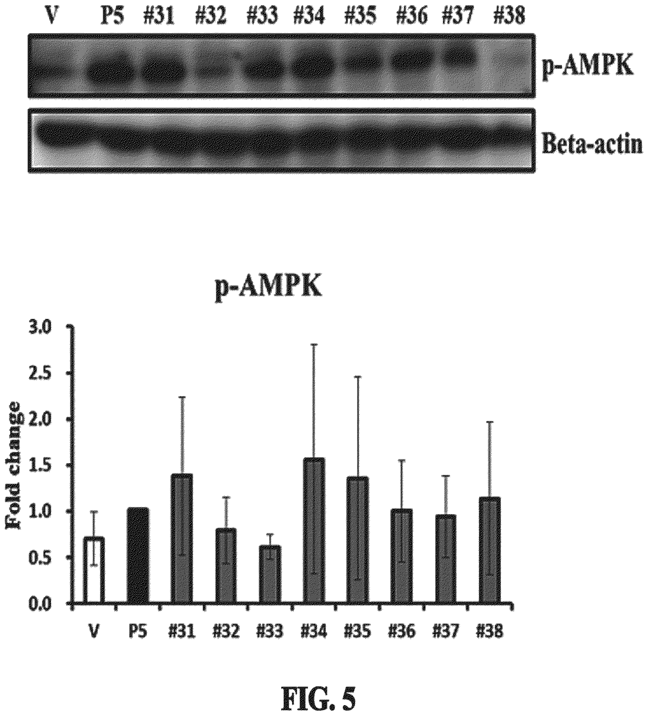

FIG. 5 is a graph showing effects of peptide derivatives DS-35 to DS-38 of the present disclosure represented by [Chemical Formula 1-21] to [Chemical Formula 1-24] on AMPK phosphorylation activity in mouse adipocytes. At this time, V is a vehicle treated with DMSO, and P5 is an APN5 peptide as a positive control and has a sequence NH₂-GLYYF-OH.

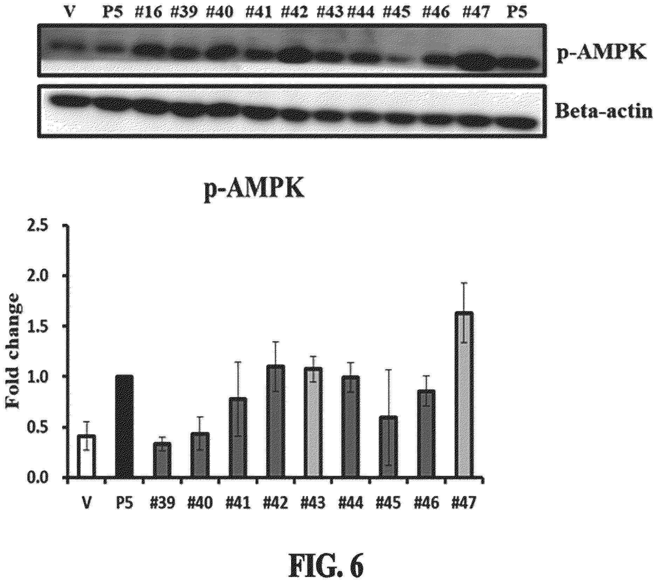

FIG. 6 is a graph showing effects of peptide derivatives DS-39 to DS-42, DS-44, and DS-45 of the present disclosure represented by [Chemical Formula 1-25] to [Chemical Formula 1-30] on AMPK phosphorylation activity in mouse adipocytes. At this time, V is a vehicle treated with DMSO, and P5 is an APN5 peptide as a positive control and has a sequence NH₂-GLYYF-OH.

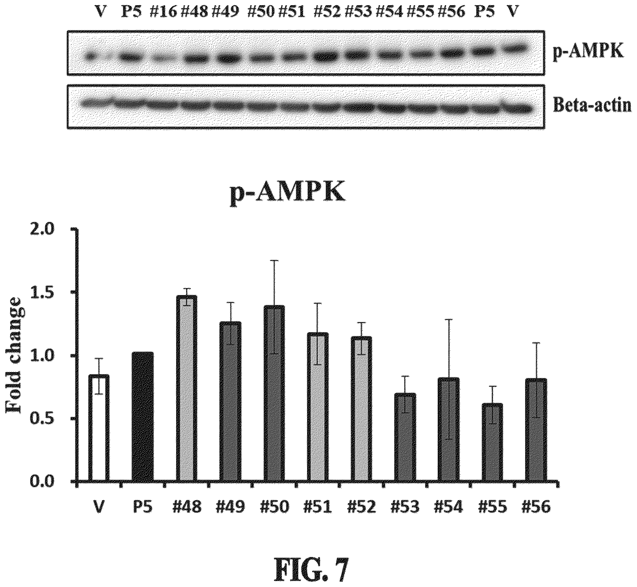

FIG. 7 is a graph showing effects of peptide derivatives DS-48 to DS-56 of the present disclosure represented by [Chemical Formula 1-31] to [Chemical Formula 1-39] on AMPK phosphorylation activity in mouse adipocytes. At this time, V is a vehicle treated with DMSO, and P5 is an APN5 peptide as a positive control and has a sequence NH₂-GLYYF-OH.

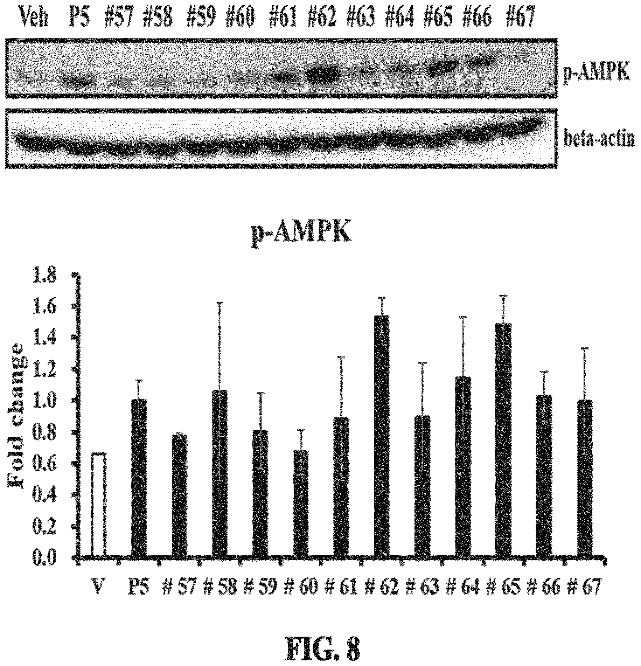

FIG. 8 is a graph showing effects of peptide derivatives DS-57 to DS-63 of the present disclosure represented by [Chemical Formula 1-40] to [Chemical Formula 1-46] on AMPK phosphorylation activity in mouse adipocytes. At this time, V is a vehicle treated with DMSO, and P5 is an APN5 peptide as a positive control and has a sequence NH₂-GLYYF-OH.

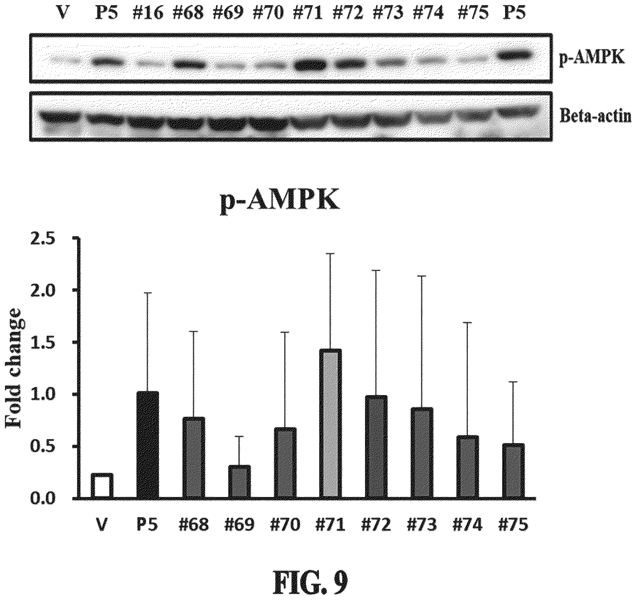

FIG. 9 is a graph showing effects of peptide derivatives DS-68, DS-70, and DS-72 to DS-75 of the present disclosure represented by [Chemical Formula 1-47] to [Chemical Formula 1-52] on AMPK phosphorylation activity in mouse adipocytes. At this time, V is a vehicle treated with DMSO, and P5 is an APN5 peptide as a positive control and has a sequence NH₂-GLYYF-OH.

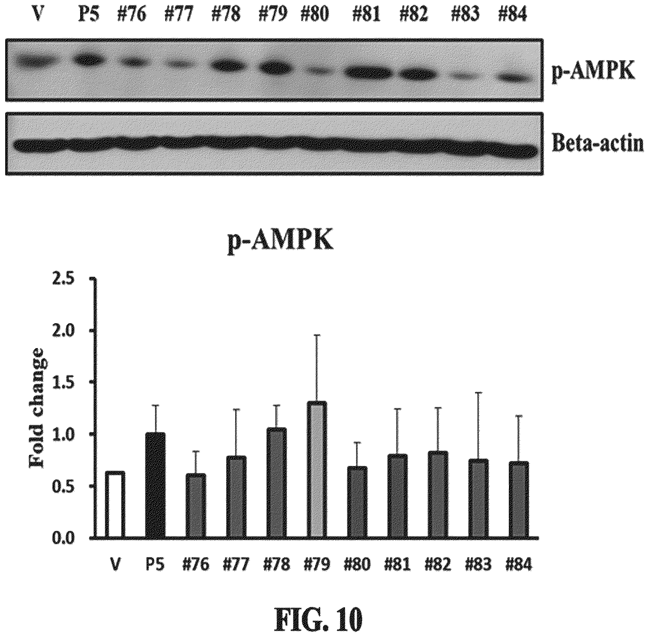

FIG. 10 is a graph showing effects of peptide derivatives DS-76 to DS-79 of the present disclosure represented by [Chemical Formula 1-53] to [Chemical Formula 1-56] on AMPK phosphorylation activity in mouse adipocytes. At this time, V is a vehicle treated with DMSO, and P5 is an APN5 peptide as a positive control and has a sequence NH₂-GLYYF-OH.

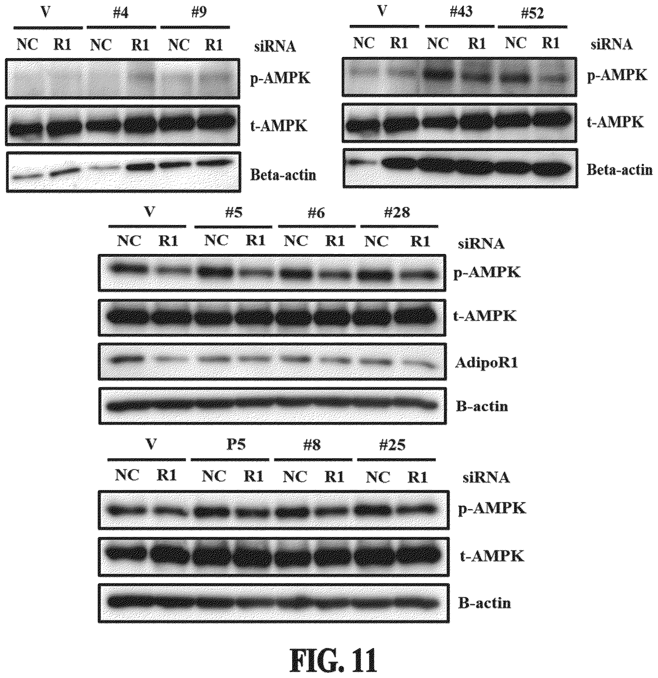

FIG. 11 illustrates whether the AMPK phosphorylation activity that had increased by the peptide derivatives DS-4, DS-5, DS-6, DS-8, DS-28, DS-43, and DS-52 of the present disclosure represented by [Chemical Formula 1-4], [Chemical Formula 1-5], [Chemical Formula 1-6], [Chemical Formula 1-8], [Chemical Formula 1-9], [Chemical Formula 1-18], and [Chemical Formula 1- 35] is reduced when an adiponectin receptor 1 is knocked down. At this time, NC is scrambled siRNA, and R1 is adiponectin receptor 1 siRNA. At this time, V is a vehicle treated with DMSO, and P5 is an APN5 peptide as a positive control and has a sequence NH₂-GLYYF-OH. DS-43 is an adiporon, which is an agonist of the adiponectin receptor 1.

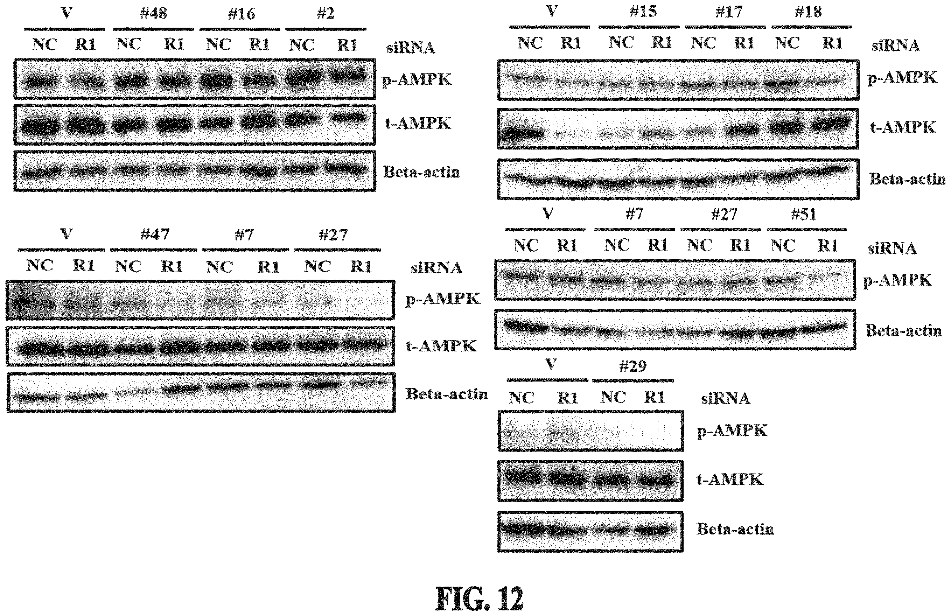

FIG. 12 illustrates whether the AMPK phosphorylation activity that had increased by the peptide derivatives DS-2, DS-7, DS-17, DS-18, DS-27, DS-29, DS-48, and DS-51 of the present disclosure represented by [Chemical Formula 1-2], [Chemical Formula 1-7], [Chemical Formula 1-13], [Chemical Formula 1-14], [Chemical Formula 1-17], [Chemical Formula 1-19], [Chemical Formula 1-31], and [Chemical Formula 1-34] is reduced when an adiponectin receptor 1 is knocked down. At this time, NC is scrambled siRNA, and R1 is adiponectin receptor 1 siRNA. At this time, V is a vehicle treated with DMSO, and P5 is an APN5 peptide as a positive control and has a sequence NH₂-GLYYF-OH. DS-43 is an adiporon, which is an agonist of the adiponectin receptor 1.

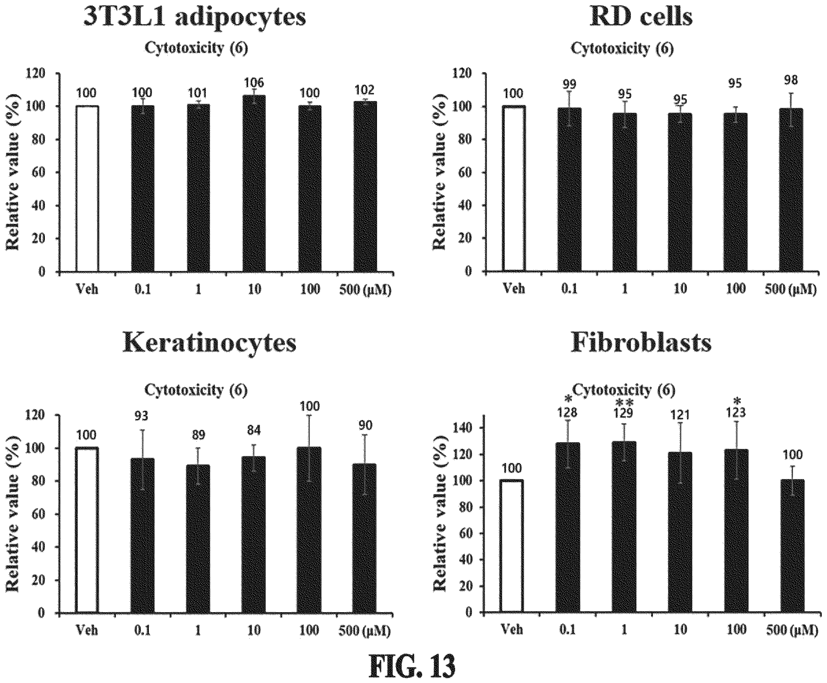

FIG. 13 illustrates confirming cytotoxicity of a peptide derivative of the present disclosure represented by [Chemical Formula 1-6] for four cell lines 3T3-L1 adipocytes, RD muscle cells, keratinocytes, and fibroblasts. At this time, Veh is a vehicle treated with DMSO.

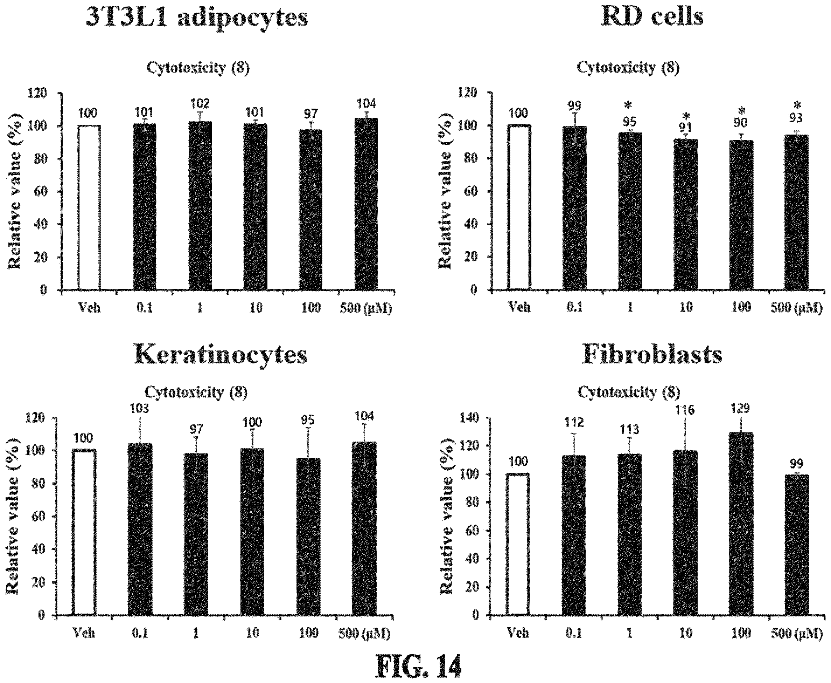

FIG. 14 illustrates confirming cytotoxicity of a peptide derivative of the present disclosure represented by [Chemical Formula 1-8] for four cell lines 3T3-L1 adipocytes, RD muscle cells, keratinocytes, and fibroblasts. At this time, Veh is a vehicle treated with DMSO.

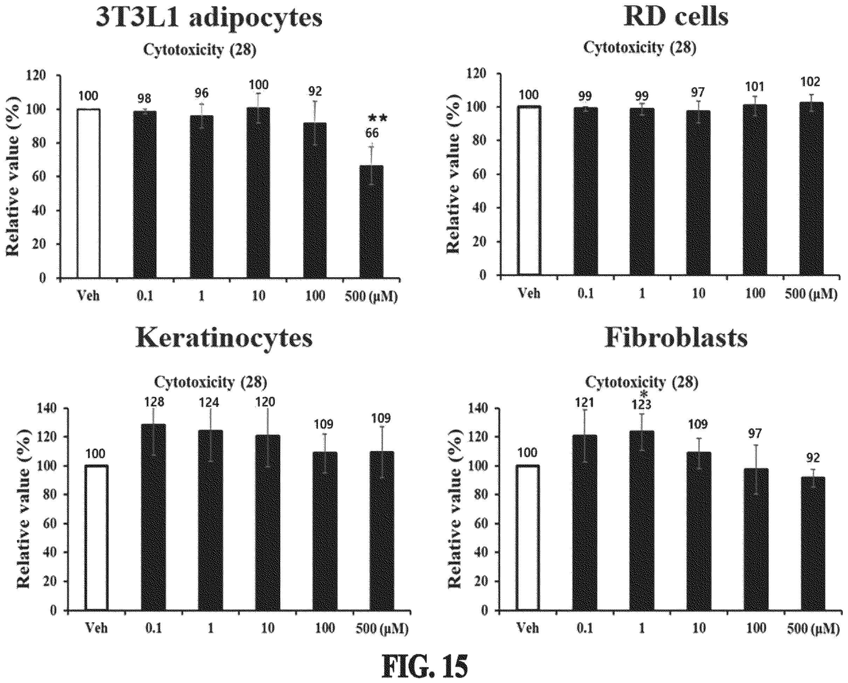

FIG. 15 illustrates confirming cytotoxicity of a peptide derivative of the present disclosure represented by [Chemical Formula 1-18] for four cell lines 3T3-L1 adipocytes, RD muscle cells, keratinocytes, and fibroblasts. At this time, Veh is a vehicle treated with DMSO.

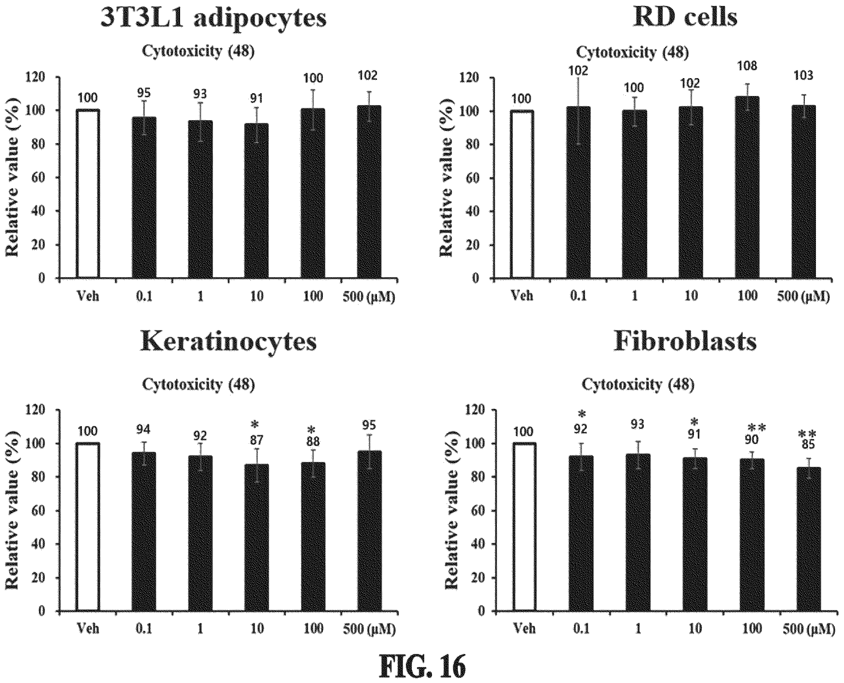

FIG. 16 illustrates confirming cytotoxicity of a peptide derivative of the present disclosure represented by [Chemical Formula 1-31] for four cell lines 3T3-L1 adipocytes, RD muscle cells, keratinocytes, and fibroblasts. At this time, Veh is a vehicle treated with DMSO.

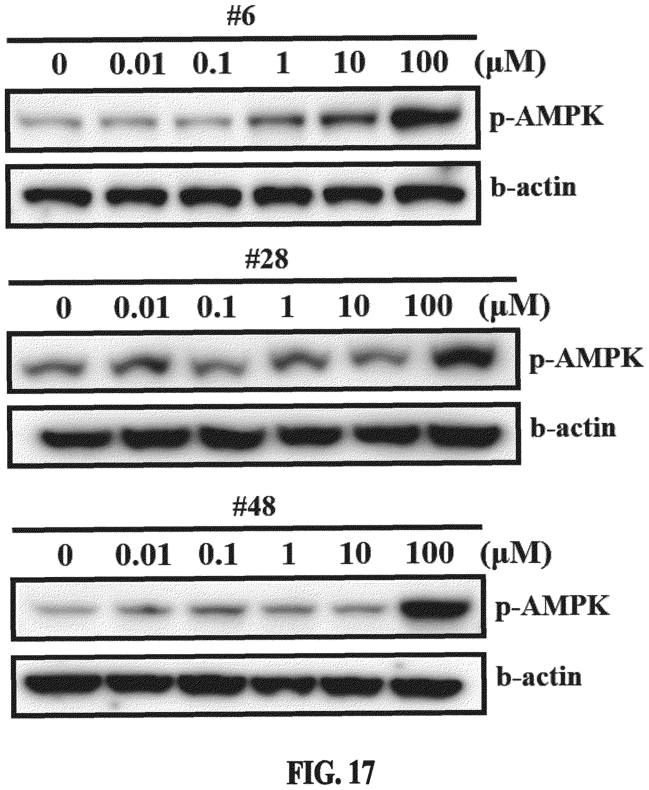

FIG. 17 illustrates whether peptide derivatives of the present disclosure represented by [Chemical Formula 1-6], [Chemical Formula 1-18], and [Chemical Formula 1-31] increase AMPK phosphorylation activity in a concentration-dependent manner.

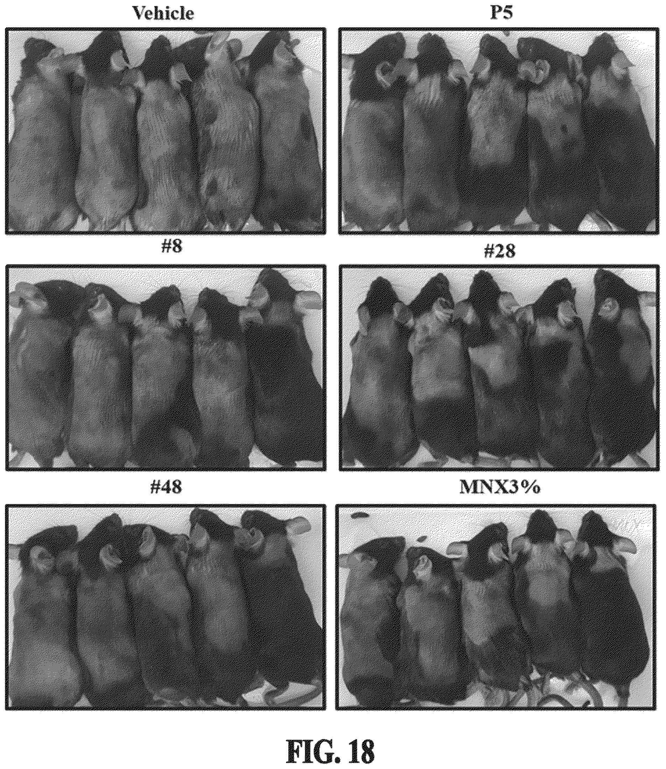

FIG. 18 illustrates hair growth efficacy of peptide derivatives of the present disclosure represented by [Chemical Formula 1-8], [Chemical Formula 1-18], and [Chemical Formula 1-31]. At this time, V is a vehicle at EtOH : polyethylene glycol = 30 : 70, v/v, P5 is an APN5 peptide as a positive control and has an NH₂-GLYYF-OH sequence, and Mnx is minoxidil known as a hair growth agent as a positive control.

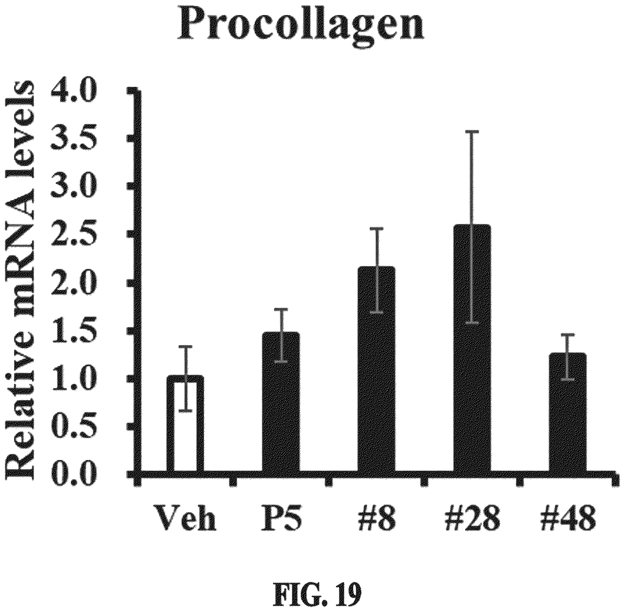

FIG. 19 illustrates skin aging alleviation efficacy of peptide derivatives of the present disclosure represented by [Chemical Formula 1-8], [Chemical Formula 1-18], and [Chemical Formula 1-31]. At this time, Veh is a vehicle treated with DMSO and P5 is an APN5 peptide as a positive control.

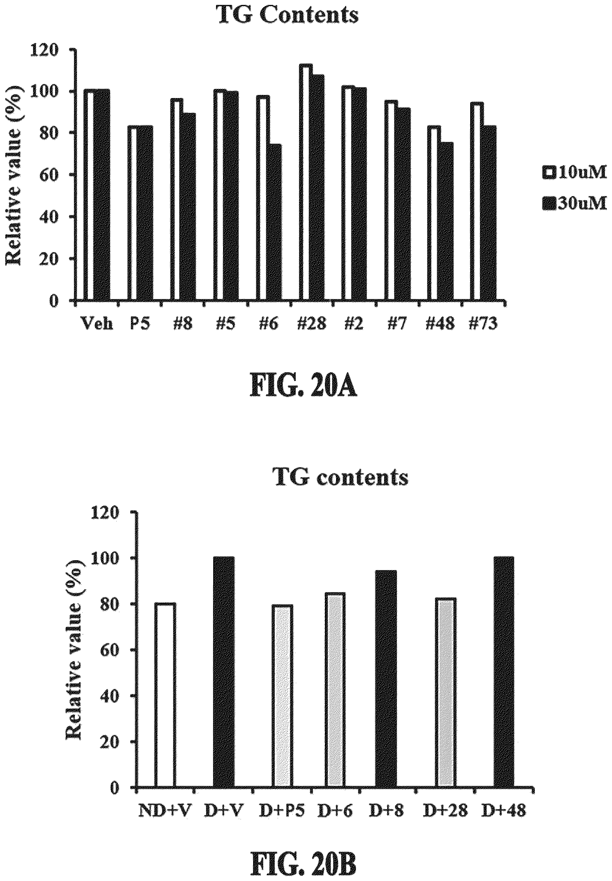

FIG. 20A illustrates neutral fat removal efficacy of peptide derivatives of the present disclosure represented by [Chemical Formula 1-8], [Chemical Formula 1-5], [Chemical Formula 1-6], [Chemical Formula 1-18], [Chemical Formula 1-2], [Chemical Formula 1-7], [Chemical Formula 1-31] and [Chemical Formula 1-50]. FIG. 20B illustrates differentiation and neutral fat inhibition efficacy of peptide derivatives of the present disclosure represented by [Chemical Formula 1-6], [Chemical Formula 1-8], [Chemical Formula 1-18], and [Chemical Formula 1-31]. At this time, V is a vehicle treated with DMSO, and P5 is an APN5 peptide as a positive control and has a sequence NH₂-GLYYF-OH. ND was cultured in a non-differentiation medium, and D is cultured in a differentiation medium.

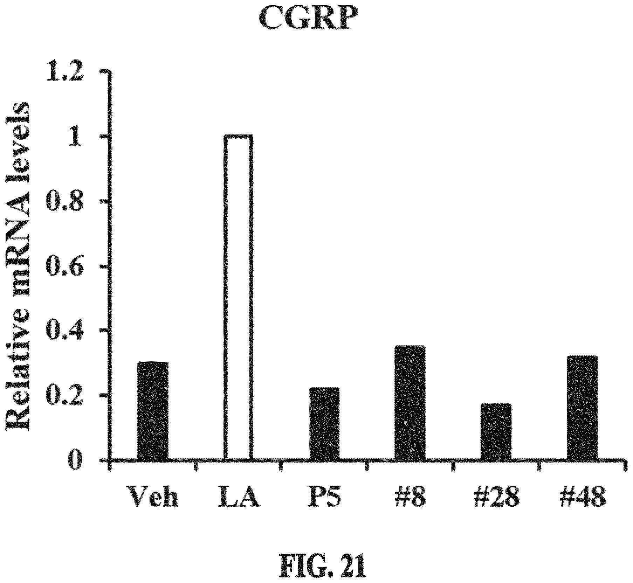

FIG. 21 illustrates sensitive skin alleviation efficacy of peptide derivatives of the present disclosure represented by [Chemical Formula 1-8], [Chemical Formula 1-18], and [Chemical Formula 1-31]. At this time, Veh is a vehicle treated with DMSO, LA is lactic acid, and P5 is an APN5 peptide as a positive control.

### BEST MODE FOR CARRYING OUT THE INVENTION

The present disclosure relates to the synthesis of a novel peptide derivative having an adiponectin-derived peptide APN5 as a matrix, and more specifically, to a peptide in which some amino acids have been modified to improve the physical properties and activity of conventional adiponectin peptides. The peptide derivative of the present disclosure has improved efficacy such as physical properties and activity, and thus is advantageous for formulation of a drug.

Therefore, the present disclosure provides a peptide derivative represented by [Chemical Formula 1] below.

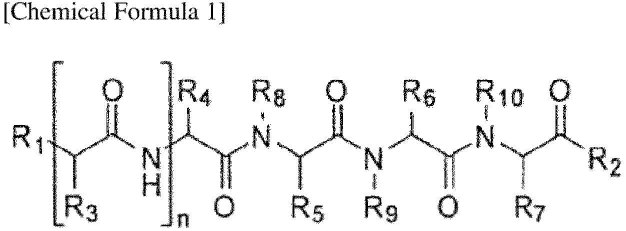

In [Chemical Formula 1],
R₁ is at least one selected from the group consisting of an amino group (NH₂), an acetylamino group (Ac-NH), NH₂-(CH₂CH₂O)ₘ-CH₂CONH, a C₁₅-C₂₀ amide group, and combinations thereof, in which m may be an integer of 1 to 5,
R₂ is an amino group or a hydroxyl group (OH),
R₃ is hydrogen (H),
R₄ is at least one selected from the group consisting of a C₁-C₆ acyclic alkyl group, a C₂-C₆ alkylcarboxyl group, a C₁-C₆ alkylamide group, an imidazoylmethyl group, and combinations thereof,
R₅ and R₆ are the same as or different from each other, and are each independently a benzyl group, a phenylethyl group, or an indolylmethyl group, in which the benzyl group is unsubstituted or at least one selected from the group consisting of a hydroxyl group, a trifluoromethyl group (CF₃), a halogen group, a cyano group, a nitro group, a C₁-C₆ acyclic alkyl group, a C₁-C₆ alkoxy group, and combinations thereof,
R₇ is a benzyl group or a C₁-C₆ alkylamino group, in which the benzyl group is unsubstituted or at least one selected from the group consisting of a hydroxyl group, a halogen group, a cyano group, a nitro group, a C₁-C₆ acyclic alkyl group, a C₁-C₆ alkoxy group, and combinations thereof,
R₃ and R₇ are linked to each other to form a C₁₅-C₂₀ heterocycloalkene group,
R₈, R₉, and R₁₀ are the same as or different from each other, and are each independently hydrogen or a C₁-C₆ alkyl group, and
n may be 0 or 1.

As used in the present disclosure, the term "substitution" refers to a reaction in which atoms or atom groups included in the molecules of the compound are replaced with other atoms or atom groups.

As used in the present disclosure, the term "acyclic" refers to a molecule having a chain structure, and the chain structure is a chemical structure in which carbon atoms are linked to each other in a chain shape, and has a straight chain shape and a branched shape.

As used in the present disclosure, the term "cyclic" refers to a structure in which both ends concatenated in the backbone of an organic compound are linked to each other to form a ring shape.

As used in the present disclosure, the term "acyclic or cyclic alkyl group" means a monovalent linear or branched or cyclic saturated hydrocarbon residue having 1 to 12 carbon atoms and consisting of only carbon and hydrogen atoms. Examples of such an alkyl group include methyl, ethyl, propyl, isopropyl, butyl, isobutyl, 2-butyl, 3-butyl, pentyl, n-hexyl, cyclobutyl, cyclopentyl, cyclohexyl groups, and the like, but are not limited thereto.

In the present disclosure, the term "halogen group" refers to elements belonging to Group 17 of the periodic table, and may be fluorine (F), chloride (Cl), bromine (Br), iodine (I), etc.

In the present disclosure, the term "alkoxy group" refers to an atom group CₙH₂ₙ₊₁O⁻ formed by bonding an oxygen atom to an alkyl group, and examples of such an alkoxy group include methoxy, ethoxy, propoxy, butoxy, and the like, but are not limited thereto.

In the present disclosure, the term "cycloalkene group" is also referred to as a cyclic alkene group, and refers to a ring group in which all ring members are carbon and have one or more double bonds (but are not aromatic). The term "heterocycloalkene group" refers to a cycloalkene group each containing at least one N, O, or S heteroatom. The cycloalkene group or heterocycloalkene group each includes one or more ring structures, and may be, for example, a single ring, a double ring, a triple ring, etc.

Further, the present disclosure provides a pharmaceutical composition for preventing or treating at least one selected from the group consisting of inflammatory skin diseases, wounds, hair loss, fibrosis, metabolic diseases, and cancer, including the peptide derivative as an active ingredient.

Further, the present disclosure provides a pharmaceutical composition for preventing or alleviating at least one selected from the group consisting of aging, sensitive skin, wrinkles and moisturization, including the peptide derivative as an active ingredient.

As used in the present disclosure, the term "prevention" means all actions that inhibit or delay the occurrence, spread or recurrence of the diseases by administering the composition of the present disclosure, and the "treatment" means all actions that improve or beneficially change the symptoms of the diseases by administering the composition of the present disclosure.

As used in the present disclosure, the term "alleviation" means all actions that at least reduce parameters associated with conditions to be treated, e.g., the degree of symptoms, or improve and beneficially change the diseases.

As used in the present disclosure, the term "pharmaceutical composition" means being prepared for preventing or treating the diseases, and may be formulated and used in various forms according to conventional methods. For example, the pharmaceutical composition may be prepared for oral formulations, such as powders, granules, tablets, capsules, suspensions, emulsions, syrups, and the like, and formulated and used in the form of external preparations, suppositories, and sterile injection solutions.

In the present disclosure, "included as the active ingredient" means that the corresponding ingredient is included in an amount required or sufficient to realize a desired biological effect. In actual application, the amount included as an active ingredient may be determined by considering matters that do not cause other toxicities, as an amount for treating a target disease, and for example, may vary depending on various factors, such as a disease or condition to be treated, a type of composition to be administered, the size of a subject, and the severity of the disease or condition. Effective amounts of individual compositions may be determined empirically without undue experimentation by those skilled in the art to which the present disclosure pertains.

In addition, the pharmaceutical composition of the present disclosure may further include one or more pharmaceutically acceptable carriers in addition to the above-described active ingredients according to each formulation.

The pharmaceutically acceptable carrier may be saline, sterile water, a Ringer's solution, buffered saline, a dextrose solution, a maltodextrin solution, glycerol, ethanol, and mixtures of at least one ingredient thereof, and if necessary, may also further include other conventional additives such as antioxidants, buffers, bacteriostats, etc. In addition, the pharmaceutical composition may also be prepared in injectable formulations such as aqueous solutions, suspensions, and emulsions, pills, capsules, granules, or tablets by further adding a diluent, a dispersant, a surfactant, a binder, and a lubricant. Furthermore, the pharmaceutical composition may also be desirably prepared according to each disease or ingredient by a suitable method of the art, or using a method disclosed in Remington's Pharmaceutical Science (Mack Publishing Company, Easton PA).

The composition of the present disclosure may be administered orally or parenterally in a pharmaceutically effective amount according to a desired method. As used in the present disclosure, the term "pharmaceutically effective amount" refers to an amount enough to treat the disease at a reasonable benefit/risk ratio applicable to medical treatment and not causing the side effects. The effective amount level may be determined according to factors including the health condition of a patient, the severity, the activity of a drug, the sensitivity to a drug, an administration method, a time of administration, a route of administration, an emission rate, duration of treatment, and combined or simultaneously used drugs, and other factors well-known in the medical field.

As used in the present disclosure, the "inflammatory skin disease" refers to inflammation that occurs in the outer layer of the skin, causing itching, blisters, redness, swelling, and often exudation, crusting, and peeling. Non-limiting examples thereof include psoriasis, atopic dermatitis, eczema, contact dermatitis, erythroderma, lichen simplex chronicus, dermatitis nummular, seborrheic dermatitis, stasis dermatitis, etc., but are not limited thereto.

As used in the present disclosure, the "metabolic disease" refers to a syndrome in which risk factors such as obesity, diabetes, hypertension, arteriosclerosis, and nonalcoholic fatty liver disease (NAFLD) occur due to accumulation of excess nutrients in the body and lack of exercise. Non-limiting examples thereof include diabetes, hypertension, hyperlipidemia, cerebrovascular disease, thrombosis, dyslipidemia, stroke, arteriosclerosis, hyperinsulinemia, etc., but are not limited thereto.

Further, the present disclosure provides a method for preventing or treating at least one selected from the group consisting of inflammatory skin diseases, wounds, hair loss, fibrosis, metabolic diseases, and cancer, including administering the peptide derivative to a subject.

Further, the present disclosure provides a method for preventing or alleviating at least one selected from the group consisting of aging, sensitive skin, wrinkles and moisturization, including administering the peptide derivative to a subject.

As used in the present disclosure, the term "subject" is any mammal, such as livestock or humans required for prevention, treatment, and/or diagnosis of the diseases without limitation, and may be desirably humans.

As used in the present disclosure, the term "administration" means providing a predetermined material to a patient by any appropriate method. The pharmaceutical composition of the present disclosure may be formulated in various forms for administration to a subject, and a representative formulation for parenteral administration is an injectable formulation, and desirably an isotonic aqueous solution or suspension. The injectable formulation may be prepared according to techniques known in the art by using suitable dispersing or wetting agents and suspending agents. For example, the injectable formulation may be formulated for injection by dissolving each ingredient in saline or buffer. In addition, the formulations for oral administration include, for example, ingestible tablets, buccal tablets, troches, capsules, elixirs, suspensions, syrups, wafers, and the like. In addition to the active ingredients, these formulations may include diluents (e.g., lactose, dextrose, sucrose, mannitol, sorbitol, cellulose and/or glycine) and lubricants (e.g., silica, talc, stearic acid and magnesium or calcium salts thereof and/or or polyethylene glycol). The tablets may include a binder such as magnesium aluminum silicate, starch paste, gelatin, tragacanth, methylcellulose, sodium carboxymethylcellulose and/or polyvinylpyrrolidine. In some cases, the tablets may further include a disintegrant such as starch, agar, alginic acid or sodium salt thereof, an absorbent, a coloring agent, a flavoring agent and/or a sweetening agent. The formulations may be prepared by conventional mixing, granulating or coating methods.

In addition, the pharmaceutical composition of the present disclosure may further include adjuvants such as preservatives, hydrating agents, emulsifying accelerators, and salts or buffers for osmotic pressure control, and other therapeutically useful materials, and may be formulated according to conventional methods.

The pharmaceutical composition according to the present disclosure may be administered through various routes including oral, transdermal, subcutaneous, intravenous, intranasal, intraperitoneal and intramuscular routes, and the dose of the active ingredient may be appropriately selected according to various factors such as a route of administration, the age, sex, and weight of a patient, and the severity of a patient. In addition, the composition of the present disclosure may be administered in combination with known compounds capable of increasing the desired effect.

Further, the present disclosure provides a cosmetic composition for preventing or alleviating at least one selected from the group consisting of inflammatory skin diseases, wounds, hair loss, fibrosis, metabolic diseases, and cancer, including the peptide derivative as an active ingredient.

Further, the present disclosure provides a cosmetic composition for preventing or alleviating at least one selected from the group consisting of aging, sensitive skin, wrinkles and moisturization, including the peptide derivative as an active ingredient.

The cosmetic composition includes, for example, the peptide derivative or a cosmetically acceptable salt thereof as an active ingredient, and may be prepared in the form of a basic cosmetic composition (lotion, cream, essence, face cleanser such as cleansing foam and cleansing water, packs, and body oils), a colored cosmetic composition (foundation, lipstick, mascara, and makeup base), a hair product composition (shampoo, conditioner, hair conditioner, and hair gel), soap, etc., in addition to dermatologically acceptable excipients.

The excipients may include, for example, skin emollients, skin penetration enhancers, colorants, fragrances, emulsifiers, thickeners and solvents, and more specifically starch, glucose, lactose, sucrose, gelatin, malt, rice, wheat flour, chalk, silica gel, sodium stearate, glycerol monostearate, talc, sodium chloride, anhydrous skim milk, glycerol, propylene, glycol, water, ethanol, etc., but are not limited thereto.

Further, the present disclosure provides a food composition for preventing or alleviating at least one selected from the group consisting of inflammatory skin diseases, wounds, hair loss, fibrosis, metabolic diseases, and cancer, including the peptide derivative as an active ingredient.

Further, the present disclosure provides a food composition for preventing or alleviating at least one selected from the group consisting of aging, sensitive skin, wrinkles and moisturization, including the peptide derivative as an active ingredient.

The food composition includes, for example, the peptide derivative or a pharmaceutically acceptable salt thereof as an active ingredient, and when the peptide derivative is used as an additive to the food composition, the peptide derivative may be added as it is or used with other foods or food ingredients, and may be used appropriately according to conventional methods. In general, when preparing foods or beverages, the composition of the present disclosure is added in an amount of 15 wt% or less, desirably 10 wt% or less, based on the raw material. However, in the case of long-term intake for the purpose of health and hygiene or for the purpose of health control, the amount of the active ingredients may be equal to or less than the range, and since there is no problem in terms of safety, the active ingredients may be used even in an amount above the range. That is, the mixing amount of the active ingredients may be appropriately determined depending on each purpose of use, such as prevention, health, or treatment.

The formulations of the food composition are able to be in the form of powders, granules, pills, tablets, capsules, as well as general foods or beverages.

The food of the present disclosure is able to be prepared by methods which are commonly used in the art and may be prepared by adding raw materials and ingredients which are commonly added in the art in the preparation. Specifically, the food may include proteins, carbohydrates, fats, nutrients, seasonings, and flavoring agents, and examples of the carbohydrates include glucose, fructose, maltose, sucrose, oligosaccharides, dextrin, cyclodextrin, xylitol, sorbitol, erythritol, saccharin, or synthetic flavoring agents, but are not limited thereto.

The terms used in the examples are used for the purpose of description only, and should not be construed to be limited. The singular expression includes the plural expression unless the context clearly dictates otherwise. In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present disclosure.

In describing the components of the examples of the present disclosure, terms including first, second, A, B, (a), (b), and the like may be used. These terms are just intended to distinguish the components from other components, and the terms do not limit the nature, sequence, or order of the components. When it is disclosed that any component is "connected", "bound", or "linked" to other components, it should be understood that the component may be directly connected or linked to other components, but another component may be "connected", "bound", or "linked" between the respective components.

Hereinafter, Examples will be described in detail with reference to the accompanying drawings. However, since various modifications may be made to the Examples, the scope of the present disclosure is not limited or restricted by these Examples. It should be understood that all modifications, equivalents and substitutes for Examples are included in the scope of the present disclosure.

In addition, in the description with reference to the accompanying drawings, like components designate like reference numerals regardless of reference numerals and a duplicated description thereof will be omitted. In describing the Examples, a detailed description of related known technologies will be omitted if it is determined that they unnecessarily make the gist of the Examples unclear.

The present disclosure may have various modifications and various Examples, and specific Examples will be hereinafter illustrated in the drawings and described in detail in the detailed description. However, the present disclosure is not limited to specific Embodiments, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### MODE FOR CARRYING OUT THE INVENTION

### Example 1. Preparation of peptide derivative of the present disclosure

A peptide derivative of the present disclosure was prepared using a conventional amino acid synthesis method (Umbarger. H. E., Ann. Rev. Biochem. 1978, 47, 533-606).

The start of a C-terminal was prepared to a peptide consisting of a general C-terminal as a COOH carboxyl group using a Wang resin (100-200 mesh, Novabiochem^{®}, CAS No: 65307-53-1) and a peptide consisting of a C-terminal as a CONH2 amide terminal using a Rink amide AM resin (100-200 mesh, Novabiochem^{®}, CAS No: 65307-53-1).

### Example 1.1. In the case of amino group at N-terminal and carboxyl group at C-terminal

To prepare a peptide consisting of a carboxyl group at a C-terminal, a dried Wang resin (100 mg, 0.5 to 1.3 mmol/g) was stirred for 60 minutes in a dichloromethane (DCM) solvent by using a polypropylene syringe from Torviq Co., Ltd. to remove the solvent and then a reaction in a first step started. In the reaction in the first step, 1-hydroxybenzotriazole (HOBt), N,N'-diisopropylcarbodiimide (DIC), 4-dimethylaminopyridine (DMAP) and a first amino acid residue, Fmoc-Phe-OH, were stirred for 2 hours in a solvent of dichloromethane (DCM) and dimethylformamide (DMF) at a ratio of 3 : 2, and then the washing process was repeated twice each using dimethylformamide (DMF), methyl alcohol (MeOH), dichloromethane (DCM), and final dimethylformamide (DMF), respectively, to wash the resin. In peptide monomer synthesis in a second step, for a Fmoc protecting group removal reaction, twice reaction was performed for each 10 minutes at room temperature using 1 mL of dimethylformamide (DMF) containing 20% (v/v) piperidine and then the washing process was repeated twice each using dimethylformamide (DMF), methyl alcohol (MeOH), dichloromethane (DCM), and final dimethylformamide (DMF), respectively, to wash the resin. Thereafter, 1-hydroxybenzotriazole (HOBt), N-[(dimethylamino-)-1H-1,2,3-triazolo[4,5-b]pyridin-1-yl-methylene]-N-methylmethanaminium hexafluorophosphate N-oxide (HATU), N,N-diisopropylethylamine (DIEA) and Fmoc-Tyr(OtBu)-OH were reacted for 2 hours in dimethylformamide (DMF) solvent and stirred. After the reaction was completed, the washing process of the resin was repeated twice each using dimethylformamide (DMF), methyl alcohol (MeOH), dichloromethane (DCM), and final dimethylformamide (DMF). Peptide monomer synthesis in the following steps was also performed through the same process as the second monomer synthesis step. The peptide for which the monomer synthesis was completed for each step was finally subjected to a reaction to remove the Fmoc protecting group of the last peptide of the resin, using 1 mL of dimethylformamide (DMF) with 20% (v/v) piperidine added for 10 minutes at room temperature. After reacting twice for each minute, the washing process was repeated twice each using dimethylformamide (DMF), methyl alcohol (MeOH), dichloromethane (DCM), and finally dimethylformamide (DMF) to complete the reaction. The peptide was purified using high-performance liquid chromatography (Prep-HPLC, Gilson) using a gradient solvent composition of acetonitrile and water. To confirm and analyze purity, a peptide with a purity of 98% or higher was obtained using RP-HPLC and LC-MS.

### Example 1.2. In the case of amino group at N-terminal and amide group at C-terminal

To prepare a peptide consisting of an amide group at a C-terminal, a dried Rink amide AM resin (100 mg, 0.9 mmol/g) was stirred for 60 minutes in a dimethylformamide (DMF) solvent by using a reaction vessel polypropylene syringe from Torviq Co., Ltd. to remove the solvent and then a reaction in a first step started. For a Fmoc protecting group removal reaction from the resin, the Fmoc-Rink amid MBHA resin was twice reacted for each 10 minutes at room temperature using 1 mL of dimethylformamide (DMF) containing 20% (v/v) piperidine and then the washing process was repeated twice each using dimethylformamide (DMF), methyl alcohol (MeOH), dichloromethane (DCM), and final dimethylformamide (DMF), respectively, to wash the resin. Thereafter, to introduce a first amino acid residue, 1-hydroxybenzotriazole (HOBt), N-[(dimethylamino-)-1H-1,2,3-triazolo[4,5-b]pyridin-1-yl-methylene]-N-methylmethanaminium hexafluorophosphate N-oxide (HATU), N,N-diisopropylethylamine (DIEA) and Fmoc-Phe-OH were reacted for 2 hours in a dimethylformamide (DMF) solvent and stirred. After the reaction was completed, the washing process of the resin was repeated twice each using dimethylformamide (DMF), methyl alcohol (MeOH), dichloromethane (DCM), and final dimethylformamide (DMF). In peptide monomer synthesis in a second step, for a Fmoc protecting group removal reaction, twice reaction was performed for each 10 minutes at room temperature using 1 mL of dimethylformamide (DMF) containing 20% (v/v) piperidine and then the washing process was repeated twice each using dimethylformamide (DMF), methyl alcohol (MeOH), dichloromethane (DCM), and final dimethylformamide (DMF), respectively, to wash the resin. Thereafter, 1-hydroxybenzotriazole (HOBt), N-[(dimethylamino-)-1H-1,2,3-triazolo[4,5-b]pyridin-1-yl-methylene]-N-methylmethanaminium hexafluorophosphate N-oxide (HATU), N,N-diisopropylethylamine (DIEA) and Fmoc-Tyr(OtBu)-OH were reacted for 2 hours in dimethylformamide (DMF) solvent and stirred to complete the reaction. After the reaction was completed, the washing process of the resin was repeated twice each using dimethylformamide (DMF), methyl alcohol (MeOH), dichloromethane (DCM), and final dimethylformamide (DMF). Peptide monomer synthesis in the following steps was also performed through the same process as the monomer synthesis step. Finally, as an Fmoc protecting group removal reaction of the final peptide of the resin, the reaction was performed twice for each 10 minutes at room temperature using 1 mL of dimethylformamide (DMF) containing 20% (v/v) piperidine, and then the washing process was repeated twice each using dimethylformamide (DMF), methyl alcohol (MeOH), dichloromethane (DCM), and final dimethylformamide (DMF) to complete the reaction. The peptide was purified using high-performance liquid chromatography (Prep-HPLC, Gilson) using a gradient solvent composition of acetonitrile and water. To confirm and analyze purity, a peptide with a purity of 98% or higher was obtained using RP-HPLC and LC-MS.

### Example 1.3. Acetyl protection of N-terminal

Acetic anhydride and N,N-diisopropylethylamine (DIEA) were reacted with the unprotected N-terminal peptide synthesized in Example 1.1 or 1.2 and stirred in a dimethylformamide (DMF) solvent for 2 hours to complete the reaction. After the reaction was completed, the washing process of the resin was repeated twice each using dimethylformamide (DMF), methyl alcohol (MeOH), dichloromethane (DCM), and final dimethylformamide (DMF) to synthesize a peptide in which the N-terminal was protected with an acetyl group. The peptide was purified using high-performance liquid chromatography (Prep-HPLC, Gilson) using a gradient solvent composition of acetonitrile and water. To confirm and analyze purity, a peptide with a purity of 98% or higher was obtained using RP-HPLC and LC-MS.

### Example 1.4. PAL protection of N-terminal

For synthesis of a palmitic acid (PAL)-protected peptide in the N-terminal unprotected peptide synthesized in Example 1.1 or 1.2, 1-hydroxybenzotriazole (HOBt), N-[(dimethylamino-)-1H-1,2,3-triazolo[4,5-b]pyridin-1-yl-methylene]-N-methylmethanaminium hexafluorophosphate N-oxide (HATU), N,N-diisopropylethylamine (DIEA) and 2-(2-(2-(2-aminoethoxy)ethoxy)ethoxy)acetic acid (PEG) or palmitic acid (PAL) were reacted and stirred for 2 hours in a dimethylformamide (DMF) solvent. After the reaction was completed, the washing process of the resin was repeated twice each using dimethylformamide (DMF), methyl alcohol (MeOH), dichloromethane (DCM), and final dimethylformamide (DMF).

The crude peptide obtained by the method was added in a separation solution of trifluoroacetic acid (TFA) : H₂O : triisopropylsilane (TIPS) (95 : 2.5 : 2.5 vol./vol.) and reacted for 2 to 3 hours to remove the protecting group and separate the peptide from the resin, and then the peptide was precipitated with cooled diethyl ether and obtained. The peptide was purified using high-performance liquid chromatography (Prep-HPLC, Gilson) using a gradient solvent composition of acetonitrile and water. To confirm and analyze purity, a peptide with a purity of 98% or higher was obtained using RP-HPLC and LC-MS.

### Example 1.5. PEG protection of N-terminal

For synthesis of a peptide protected with (PEG)3(2-(2-(2-(2-aminoethoxy)ethoxy)ethoxy)acetic acid in the N-terminal unprotected peptide synthesized in Example 1.1 or 1.2, 1-hydroxybenzotriazole (HOBt), N-[(dimethylamino-)-1H-1,2,3-triazolo[4,5-b]pyridin-1-yl-methylene]-N-methylmethanaminium hexafluorophosphate N-oxide (HATU), N,N-diisopropylethylamine (DIEA) and 2-(2-(2-(2-aminoethoxy)ethoxy)ethoxy)acetic acid (PEG) or palmitic acid (PAL) were reacted and stirred for 2 hours in a dimethylformamide (DMF) solvent to complete the reaction. After the reaction was completed, the washing process of the resin was repeated twice each using dimethylformamide (DMF), methyl alcohol (MeOH), dichloromethane (DCM), and final dimethylformamide (DMF).

The crude peptide obtained by the method was added in a separation solution of trifluoroacetic acid (TFA) : H₂O : triisopropylsilane (TIPS) (95 : 2.5 : 2.5 vol./vol.) and reacted for 2 to 3 hours to remove the protecting group and separate the peptide from the resin, and then the peptide was precipitated with cooled diethylether and obtained. The peptide was purified using high-performance liquid chromatography (Prep-HPLC, Gilson) using a gradient solvent composition of acetonitrile and water. To confirm and analyze purity, a peptide with a purity of 98% or higher was obtained using RP-HPLC and LC-MS.

### Example 1.6. Cyclic peptide synthesis

A peptide was synthesized using a method of preparing a peptide consisting of consisting of an amide group at a C-terminal by the method of Example 1.2. The first and last amino acids were synthesized as a linear peptide in the same manner as in Example 1.2 using Fmoc-Allyl-Gly-OH, and then the resin in which the N-terminal was protected with Fmoc was added with LiCl (0.4 M) dissolved in dimethylformamide (DMF) and a Grubbs' catalyst in the dichloromethane (DCM) solvent and reacted at 100°C for 1 to 2 hours using a microwave (2.5 GHz, 300 W). After the reaction was completed, the washing process of the resin was repeated twice each using dimethylformamide (DMF), methyl alcohol (MeOH), dichloromethane (DCM), and final dimethylformamide (DMF). Finally, as an Fmoc protecting group removal reaction of the final peptide of the resin, the reaction was performed twice for each 10 minutes at room temperature using 1 mL of dimethylformamide (DMF) containing 20% (v/v) piperidine, and then the washing process was repeated twice each using dimethylformamide (DMF), methyl alcohol (MeOH), dichloromethane (DCM), and final dimethylformamide (DMF) to complete the reaction. The crude peptide obtained by the method was added in a separation solution of trifluoroacetic acid (TFA) : H₂O : triisopropylsilane (TIPS) (95 : 2.5 : 2.5 vol./vol.) and reacted for 2 to 3 hours to remove the protecting group and separate the peptide from the resin, and then the peptide was precipitated with cooled diethylether and obtained. The peptide was purified using high-performance liquid chromatography (Prep-HPLC, Gilson) using a gradient solvent composition of acetonitrile and water. To confirm and analyze purity, a peptide with a purity of 98% or higher was obtained using RP-HPLC and LC-MS (J. Pept. Sci. 2007; 13: 280-285).

The sequence and molecular weight of the peptide derivative according to Example of the present disclosure were shown in Table 3 below.

**[Table 3]**

| **Compound No.** | **Chemical Formula No.** | **Sequence** | **Measured molecular weight [M+H]⁺** |
|---|---|---|---|
| DS-1 | 1-1 | NH2-G1YYF-OH | 662.25 |
| DS-2 | 1-2 | AcNH-GlYYF-OH | 661.25 |
| DS-3 | 1-3 | NH2-G1YYF-NH2 | 703.24 |
| DS-4 | 1-4 | AcNH-GlYYF-NH2 | 664.17 |
| DS-5 | 1-5 | NH2-GDYYF-OH | 706.19 |
| DS-6 | 1-6 | AcNH-GDYYF-OH | 663.20 |
| DS-7 | 1-7 | NH2-GDYYF-NH2 | 705.21 |
| DS-8 | 1-8 | AcNH-GDYYF-NH2 | 663.20 |
| DS-9 | 1-9 | NH2-GNYYF-OH | 663.20 |
| DS-10 | 1-10 | AcNH-GNYYF-OH | 705.20 |
| DS-11 | 1-11 | NH2-GNYYF-NH2 | 662.23 |
| DS-12 | 1-12 | Ac-NH-GNYYF-NH2 | 704.22 |
| DS-17 | 1-13 | NH2-GLWYF-NH2 | 684.27 |
| DS-18 | 1-14 | IVH2-GIWYF-NH2 | 684.28 |
| DS-19 | 1-15 | IVH2-GLFYF-NH2 | 645.27 |
| DS-20 | 1-16 | NH2-GIFYF-NH2 | 645.28 |
| DS-27 | 1-17 | NH2-DYYF-OH | 607.13 |
| DS-28 | 1-18 | AcNH-DYYF-OH | 649.18 |
| DS-29 | 1-19 | NH2-DYYF-NH2 | 606.20 |
| DS-30 | 1-20 | AcNH-DYYF-NH2 | 648.25 |
| DS-35 | 1-21 | NH2-GDYYK-NH2 | 644.18 |
| DS-36 | 1-22 | AcNH-GDYYK-NH2 | 686.24 |
| DS-37 | 1-23 | NH2-GEYYK-NH2 | 658.24 |
| DS-38 | 1-24 | AcNH-GEYYK-NH2 | 700.26 |
| DS-39 | 1-25 | NH2-GLYY(Dmt)F-NH2 | 689.26 |
| DS-40 | 1-26 | NH2-GDYY(Dmt)F-NH2 | 691.23 |
| DS-41 | 1-27 | NH2-GDY(Dmt)YF-NH2 | 691.25 |
| DS-42 | 1-28 | NH2-GLY(Dmt)YF-NH2 | 689.30 |
| DS-44 | 1-29 | NH2-GHYYF-NH2 | 685.23 |
| DS-45 | 1-30 | AcNH-GHYYF-NH2 | 727.26 |
| DS-48 | 1-31 | NH2-GLYYF(N-Me)-NH2 | 675.29 |
| DS-49 | 1-32 | NH2-GLYYF(homo)-NH2 | 675.28 |
| DS-50 | 1-33 | NH2-GLF(OMe)YF-NH2 | 675.28 |
| DS-51 | 1-34 | NH2-GLF(4-F)YF-NH2 | 663.26 |
| DS-52 | 1-35 | IVH2-GLF(4-Cl)YF-NH2 | 679.23 |
| DS-53 | 1-36 | NH2-GLF(4-CN)YF-NH2 | 670.27 |
| DS-54 | 1-37 | NH2-GLF(4-NO2)YF-NH2 | 690.26 |
| DS-55 | 1-38 | NH2-GLYY(N-Me)F-NH2 | 675.26 |
| DS-56 | 1-39 | NH2-GLY(N-Me)YF-NH2 | 675.28 |
| DS-57 | 1-40 | NH2-GLF(4-Cl)YF(homo)-NH2 | 693.24 |
| DS-58 | 1-41 | IVH2-GDF(4-Cl)YF-NH2 | 681.17 |
| DS-59 | 1-42 | NH2-GLYF(OMe)F-NH2 | 675.27 |
| DS-60 | 1-43 | NH2-GLYF(4-F)F-NH2 | 663.26 |
| DS-61 | 1-44 | IVH2-GLYF(4-Cl)F-NH2 | 679.24 |
| DS-62 | 1-45 | IVH2-GLYF(4-CN)F-NH2 | 670.26 |
| DS-63 | 1-46 | NH2-GLYF(4-NO2)F-NH2 | 690.25 |
| DS-68 | 1-47 | NH2-GDYYF(homo)-NH2 | 677.20 |
| DS-70 | 1-48 | IVH2-GDYF(4-Cl)F-NH2 | 681.15 |
| DS-72 | 1-49 | Cyclic | 623.22 |
| DS-73 | 1-50 | Cyclic | 637.25 |
| DS-74 | 1-51 | Cyclic | 637.24 |
| DS-75 | 1-52 | Cyclic | 651.27 |
| DS-76 | 1-53 | AcNH-GDY(N-Me)YY-NH2 | 735.21 |
| DS-77 | 1-54 | AcNH-GDYYY(N-Me)-NH2 | 735.30 |
| DS-78 | 1-55 | PAL-NH-GDYYF-NH2 | 901.45 |
| DS-79 | 1-56 | NH2-(PEG)3-NH-GDYYF-NH2 | 852.28 |

In Table 3, each amino acid constituting the peptide derivative of the present disclosure may have the following structure.

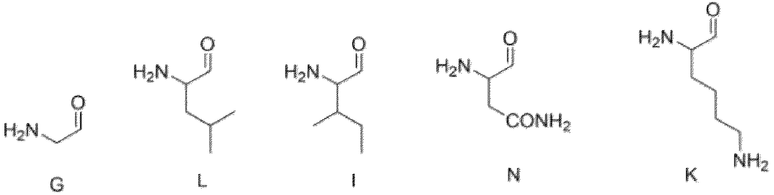

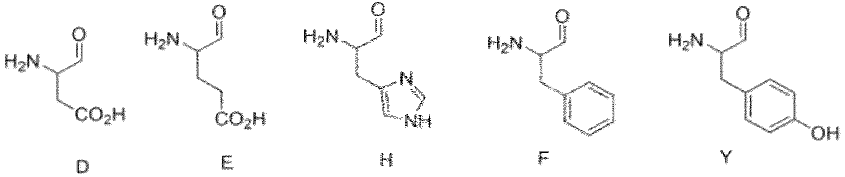

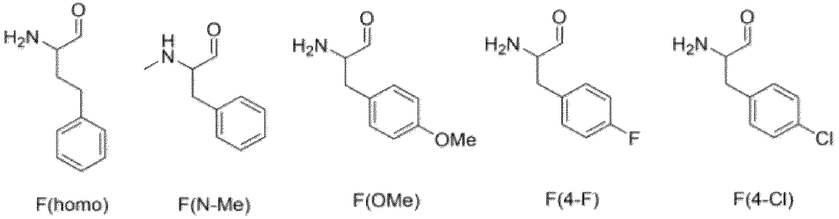

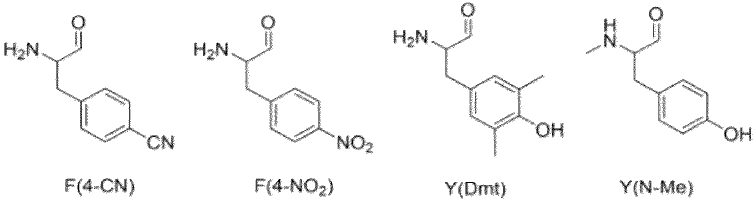

### Experimental Example 1. Verification of AMPK phosphorylation activity in vitro

There were confirmed effects of the peptide derivatives of the present disclosure on the expression and receptor activity of adiponectin in mouse adipocytes as a main adiponectin production site. A mouse adipocyte cell line 3T3-L1 was treated with each of the peptide derivatives of the present disclosure at a concentration of 10 µM and cultured for 24 hours, and then cells were obtained. Thereafter, the proteins were extracted and the effect on AMPK phosphorylation, which was responsible for the main downstream signaling of adiponectin, was confirmed by Western blot.

As a result, the peptide derivatives of the present disclosure increased AMPK phosphorylation activity associated with the activity of an adiponectin receptor in mouse adipocytes (FIGS. 1 to 10). Particularly, it was confirmed that peptide derivatives represented by [Chemical Formula 1-2], [Chemical Formula 1-4] to [Chemical Formula 1-9], [Chemical Formula 1-13], [Chemical Formula 1-14], [Chemical Formula 1-17] to [Chemical Formula 1-19], [Chemical Formula 1-31], [Chemical Formula 1-34], and [Chemical Formula 1-35] had a significant effect of increasing AMPK phosphorylation activity even compared to APN5, a previously known adiponectin-derived peptide (Table 4).

**[Table 4]**

| **Compound No.** | **Chemical Formula No.** | **AMPK phosphorylation activity (fold change)** |
|---|---|---|
| DS-8 | 1-8 | 2.6 |
| DS-5 | 1-5 | 2.0 |
| DS-6 | 1-6 | 1.9 |
| DS-28 | 1-18 | 1.9 |
| DS-4 | 1-4 | 1.8 |
| DS-9 | 1-9 | 1.6 |
| DS-48 | 1-31 | 1.5 |
| DS-2 | 1-2 | 1.3 |
| DS-7 | 1-7 | 1.3 |
| DS-27 | 1-17 | 1.3 |
| DS-51 | 1-34 | 1.3 |
| DS-17 | 1-13 | 1.2 |
| DS-18 | 1-14 | 1.2 |
| DS-29 | 1-19 | 1.2 |
| DS-52 | 1-35 | 1.2 |
| DS-43 | adiporon | 1.2 |
| APN5 | P5 | 1.0 |

### Experimental Example 2. Verification whether to lose AMPK phosphorylation activity after knockdown of adiponectin receptor 1 in vitro

In order to verify whether the peptide derivative of the present disclosure bound to an adiponectin receptor 1, a mouse adipocyte cell line 3T3-L1 was treated with siRNA of an adiponectin receptor 1 to be knocked down, and each of the peptide derivatives of the present disclosure was treated at 10 µM and cultured for 24 hours, and then cells were obtained. Thereafter, the proteins were extracted and it was confirmed by Western blot whether AMPK phosphorylation activity, which had been increased by the peptide derivatives, was lost.

As a result, it was confirmed that peptide derivatives of the present disclosure including [Chemical Formula 1-2], [Chemical Formula 1-5] to [Chemical Formula 1-8], [Chemical Formula 1-13], [Chemical Formula 1-14], [Chemical Formula 1-17] to [Chemical Formula 1-19], [Chemical Formula 1-31], [Chemical Formula 1-34], and [Chemical Formula 1-35] decreased the AMPK phosphorylation activity after knockdown of the adiponectin receptor 1 (FIGS. 11 and 12).

Adiponectin acted by binding to the adiponectin receptor, and when the adiponectin receptor was activated, it was known to increase phosphorylation of AMPK, a downstream signaling pathway, and PPARα/PPARγ activity (Crystal structures of the human adiponectin receptors. Nature. 2015 Aprl6;520(7547):312-6. doi: 10.1038/nature14301). In addition, it was known that the expression of adiponectin and its receptor was reduced in photoaged skin and UV-irradiated skin, and when the expression of adiponectin was reduced, the extracellular matrix was changed and photoaging may be deteriorated by controlling the expression of MMP-1 and procollagen, which were skin aging regulators (UV-induced inhibition of adipokine production in subcutaneous fat aggravates dermal matrix degradation in human skin.2016.Scientific Reports. 10;6:25616. doi:10.1038/srep25616).

Therefore, it is suggested that the peptide derivative of the present disclosure increases the AMPK phosphorylation activity associated with the activity of the adiponectin receptor, thereby increasing the expression of adiponectin *in vivo* and playing a major role in protecting against skin aging.

### Experimental Example 3. Verification of cytotoxicity in vitro

Using four types of cell lines 3T3-L1 adipocytes, RD muscle cells, keratinocytes, and fibroblasts, each cell was dispensed at 5 × 10⁴ cells per well in a 24-well plate and cultured for 24 hours under each cell culture condition. The medium was discarded, washed with PBS, replaced with a new medium without 10% FBS, treated with the peptide derivative of the present disclosure at different concentrations of 0, 0.1, 1, 10, 100, and 500 µM, and cultured for 24 hours. The medium was carefully removed again and washed with PBS, and then added with an MTT reagent according to a manufacturer's method, reacted at room temperature for 30 minutes, and then absorbance was measured at 450 nm.

As a result, it was confirmed that the peptide derivatives of the present disclosure including [Chemical Formula 1-6], [Chemical Formula 1-8], [Chemical Formula 1-18], and [Chemical Formula 1-31] had no toxicity in a plurality of cell lines (FIGS. 13 to 16).

### Experimental Example 4. Verification of concentration dependence of AMPK phosphorylation activity in vitro

A mouse adipocyte cell line 3T3-L1 was treated with the peptide derivative of the present disclosure at a series of concentrations of 0, 0.01, 0.1, 1, 10, and 100 µM and cultured for 24 hours, and then cells were obtained. Thereafter, the protein was extracted and the effect of the peptide derivative on AMPK phosphorylation in a concentration-dependent manner was confirmed by Western blot.

As a result, it was confirmed that the peptide derivatives of the present disclosure including [Chemical Formula 1-6], [Chemical Formula 1-18], and [Chemical Formula 1-31] increased AMPK phosphorylation activity in a concentration-dependent manner (FIG. 17).

### Experimental Example 5. Solubility Test

A PRISMA HT solution was diluted in distilled water at a ratio of 1 : 40, and then used as a test buffer by adjusting the pH to 7.4. In addition, in a general solubility test, reference materials (Diclofenac stock 50 mM, Phenazopyridine stock 25 mM) used for accuracy and the peptide derivative of the present disclosure (50 mM) as a test material were prepared in DMSO and used as a test standard solution.

10 mL of the prepared test material was diluted in 190 µL of isopropyl alcohol and prepared. In addition, 75 mL of the test solution and 70 mL of isopropyl alcohol were mixed to be used as a blank sample, and 5 mL of the test material diluted in isopropyl alcohol was added to the blank sample. After the diluted sample was mixed, the absorbance was measured and used as an initial test value. For the solubility test, 1 mL of the test solution and 10 mL of the DMSO test standard solution were mixed and then reacted at room temperature for 24 hours. Thereafter, the precipitate was removed from the sample using a filter plate, 74 mL of the filtered sample was added with 75 mL of isopropyl alcohol, and then the absorbance was measured and used as the sample value.

The solubility value of the test material was analyzed using a msol Explore (Solubility explorer program) after measuring the absorbance of each sample through a microplate reader. The average and standard deviation of the experimental values (n = 3) repeated three times were calculated using the Excel program.

As a result, the peptide derivative of the present disclosure showed excellent solubility in DMSO (Table 5).

**[Table 5]**

| **Compound No.** | **Chemical Formula No.** | **Average DMSO solubility (µg/ml)** |
|---|---|---|
| DS-2 | 1-2 | 348.0 |
| DS-5 | 1-5 | 328.0 |
| DS-6 | 1-6 | 349.0 |
| DS-8 | 1-8 | 349.0 |
| DS-27 | 1-17 | 300.0 |
| DS-28 | 1-18 | 321.0 |
| DS-29 | 1-19 | 300.0 |
| DS-43 | adiporon | 43.7 |
| DS-48 | 1-31 | 334.0 |
| DS-51 | 1-34 | 135.5 |
| DS-73 | 1-50 | 315.0 |
| APN5 | P5 | 8.2 |
| Diclofenac (reference) | | 157.0 |
| Phenazopyridine (reference) | | 23.76 |

### Experimental Example 6. Plasma stability test

The peptide derivative of the present disclosure was added at a concentration of 1 µM to two types of plasma (human, rat), placed in each tube, and then cultured at 37°C for time of 0, 30, 60, 120, and 240 minutes. The tube containing plasma was taken out every time, added with an acetonitrile solution containing an internal standard material (chlorpropamide), vortexed for 5 minutes, and centrifuged for 5 minutes (14,000 rpm, 4°C). Thereafter, the plasma stability of the peptide derivative was evaluated by injecting the supernatant into an LC-MS/MS system and analyzing the drug at each time period. At this time, Procaine and Enalapril were used as reference materials for accuracy during a general plasma stability test.

After the reaction, the amount of drug remaining in each tube was analyzed by LC-MS/MS using Shimadzu Nexera XR system and TSQ vantage (Thermo). The HPLC column was used with a Luna C18 column (2.0 × 50 mm, 3 µm particle size; Phenomenex, US), and the mobile phase was distilled water (A) containing 0.1% formic acid and acetonitrile (B) containing 0.1% formic acid. Data analysis was performed using Xcalibur (version 1.6.1). In the result analysis, the plasma stability of each peptide derivative was expressed as % remaining for each time period for the sample without culture.

As a result, it was confirmed that the peptide derivative of the present disclosure had excellent plasma stability, and in particular, compared to APN5, a conventional known adiponectin-derived peptide, the plasma stability was significantly improved (Table 6).

**[Table 6]**

| **Compound No.** | **Chemical Formula No.** | **Plasma stability (% remaining)** | | | |
|---|---|---|---|---|---|
| | | **Human, 30 m** | **Human, 120 m** | **Rat, 30 m** | **Rat, 120 m** |
| DS-5 | 1-5 | 1.4 | < 1 | > 100 | 94.1 |
| DS-6 | 1-6 | 12.6 | 1.3 | > 100 | > 100 |
| DS-8 | 1-8 | 39.8 | 3.3 | > 100 | 96 |
| DS-28 | 1-18 | 17.6 | 3.1 | > 100 | > 100 |
| DS-35 | 1-21 | 2.9 | < 1 | 99.4 | 89.4 |
| DS-41 | 1-27 | < 1 | < 1 | > 100 | 99.6 |
| DS-43 | adiporon | > 100 | > 100 | > 100 | > 100 |
| DS-48 | 1-31 | 1.1 | < 1 | > 100 | 84.9 |
| DS-56 | 1-39 | 2.8 | < 1 | 70.9 | 29.4 |
| DS-73 | 1-50 | 94.7 | 75.2 | > 100 | 89.3 |
| APN5 | P5 | < 1 | < 1 | 83.1 | 70.5 |
| Procaine (reference) | | 2.8 (5 min) | < 1 (5 min) | 90.3 | 57.4 |
| Enalapril (reference) | | > 100 | 97.4 | 27.9 | < 1 |

### Experimental Example 7. Metabolic stability test

Three types of liver microsomes (Human, Rat, Mouse, 0.5 mg/ml), a 0.1 M phosphate buffer solution (pH 7.4), and the peptide derivative of the present disclosure were added at a concentration of 1 µM and pre-cultured at 37°C for 5 minutes, and then added with an NADPH Regeneration system solution and cultured at 37°C for 30 minutes. Thereafter, to terminate the reaction, the mixture was added with an acetonitrile solution containing an internal standard material (chlorpropamide), centrifuged for 5 minutes (14,000 rpm, 4°C), and then the supernatant was injected into the LC-MS/MS system to analyze a substrate drug, and thus metabolic stability was evaluated. At this time, Verapamil was used as a reference material for accuracy during a general plasma stability test.

The amount of substrate remaining through the reaction was analyzed by LC-MS/MS using Agilent 1290 infinity series pump system (Agilent, USA) and Triple Quad 5500 LC-MS/MS system (Applied Biosystems, USA). The HPLC column was used with a Kinetex C18 column (2.1 × 100 mm, 1.7 µm particle size; Phenomenex, USA), and the mobile phase was distilled water (A) containing 0.1% formic acid and acetonitrile (B) containing 0.1% formic acid. TurboSpray Ionization was used as an ion source for MS/MS, and a triple quadruple type was used as a mass spectrometer. The produced metabolites were quantified using a multiple reaction monitoring (MRM) quantitative mode, and data analysis was performed using Analyst software (version 1.6.1).

As a result, it was confirmed that the peptide derivative of the present disclosure had excellent metabolic stability, and in particular, even compared to APN5, a conventional known adiponectin-derived peptide, the metabolic stability was significantly improved (Table 7).

**[Table 7]**

| **Compound No.** | **Chemical Formula No.** | **Metabolic stability (% remaining)** | | |
|---|---|---|---|---|
| | | **Human, 30 m** | **Rat, 30 m** | **Mouse, 30 m** |
| DS-5 | 1-5 | 62.5 | 66.4 | 95.9 |
| DS-6 | 1-6 | 88.0 | 88.1 | 96.5 |
| DS-8 | 1-8 | 93.9 | 78.2 | 83.6 |
| DS-28 | 1-18 | 93.8 | 85.8 | 96.6 |
| DS-35 | 1-21 | >100 | 95.8 | 99.2 |
| DS-41 | 1-27 | 95.8 | 75.1 | 95.2 |
| DS-43 | adiporon | 54.9 | 2.3 | 3.1 |
| DS-48 | 1-31 | 21.6 | 2.8 | 20.6 |
| DS-56 | 1-39 | 53.9 | 41.1 | 10.6 |
| DS-73 | 1-50 | 71.6 | 65.7 | 78 |
| APN5 | P5 | 1.7 | 1.3 | 9.5 |
| Verapamil (reference) | | 13.4 | - | - |

### Experimental Example 8. CYP inhibition assay

Human liver microsomes (0.25 mg/ml), a 0.1 M phosphate buffer solution (pH 7.4), a substrate drug cocktail of five drug metabolizing enzymes (Phenacetin 50 µM, S-mephenytoin 100 µM, dextromethorphan 5 µM, midazolam 2.5 µM, diclofenac 10 µM) and the peptide derivative of the present disclosure were added at concentrations of 0 and 10 µM, respectively, and pre-cultured at 37°C for 5 minutes, and then added with an NADPH Regeneration system solution and cultured at 37°C for 15 minutes. Thereafter, to terminate the reaction, the mixture was added with an acetonitrile solution containing an internal standard material (chlorpropamide), centrifuged for 5 minutes (14,000 rpm, 4°C), and then the supernatant was injected into the LC-MS/MS system to analyze metabolites of a substrate drug, and thus the inhibitory ability of drug-metabolizing enzymes by the peptide derivative was evaluated. At this time, Ketoconazole was used as a reference material used for accuracy when evaluating the inhibitory ability of general drug-metabolizing enzymes.

Metabolites of each CYP isoenzyme indicator drug produced through the reaction were analyzed by LC-MS/MS using the Shimadzu Nexera XR system and TSQ vantage (Thermo). The HPLC column was used with a Kinetex C18 column (2.1 × 100 mm, 2.6 µm particle size; Phenomenex, USA), and the mobile phase was distilled water (A) containing 0.1% formic acid and acetonitrile (B) containing 0.1% formic acid. TurboSpray Ionization was used as an ion source for MS/MS, and a triple quadruple type was used as a mass spectrometer. The produced metabolites were quantified using a multiple reaction monitoring (MRM) quantitative mode, and data analysis was performed using Xcalibur (version 1.6.1).

As a result, it is suggested that since the peptide derivative of the present disclosure inhibits CYP enzymes including CYP1A2, CYP2C9, CYP2C19, CYP2D6, and CYP3A4 (Table 8), fatal adverse reactions are not caused by drug interactions.

**[Table 8]**

| **Compound No.** | **Chemical Formula No.** | **CYP inhibition (% of control activity)** | | | | |
|---|---|---|---|---|---|---|
| | | **CYP1A2** | **CYP2C9** | **CYP2C19** | **CYP2D6** | **CYP3A4** |
| DS-5 | 1-5 | 83.2 | 97.3 | 80.9 | 85.5 | 90.9 |
| DS-6 | 1-6 | 86.1 | > 100 | 70.2 | 89.1 | 94.1 |
| DS-8 | 1-8 | 95.1 | > 100 | 100 | > 100 | > 100 |
| DS-28 | 1-18 | 81.7 | 96.9 | 87.2 | 86.1 | 95.5 |
| DS-35 | 1-21 | 77.7 | 93.6 | 4.1 | 97.4 | > 100 |
| DS-41 | 1-27 | 82.7 | 93.6 | 75.8 | 09.6 | 92.3 |
| DS-43 | adiporon | 91.6 | 57.8 | 66.0 | 30.3 | 90.3 |
| DS-48 | 1-31 | 79.0 | 85.0 | 17.0 | 78.1 | 24.1 |
| DS-56 | 1-39 | 80.5 | 100.0 | 73.5 | 91.6 | 9.2 |
| DS-73 | 1-50 | 82.7 | 96.5 | 85.1 | 85.9 | 80.5 |
| APN5 | P5 | 88.8 | > 100 | 98.5 | 92.3 | 82.9 |
| Ketoconazole (reference) | | 97.2 | > 100 | > 100 | > 100 | 26.1 |

### Experimental Example 9. Evaluation of efficacy of mouse application hair growth and alleviation of skin aging

After shaving the back of a 7-week-old C57BL/6 mouse, the peptide derivative formulations of the present disclosure were dissolved in a vehicle (EtOH : polyethylene glycol = 30 : 70, v/v) at a concentration of 0.15 mM and applied. The solution equivalent to 200 µl per mouse was applied uniformly to the entire back every day for 35 days.

In addition, after the last application, skin tissue was extracted and mRNA was extracted, and the effect on the expression of procollagen, a skin aging factor, was verified using real-time PCR.

As a result, the proportion of areas where hairs grew among areas from which hairs were first removed significantly increased in a group treated with the peptide derivative of the present disclosure. In particular, it was confirmed that formation of hair follicle was promoted similarly to a minoxidil-treated group used as a positive control group (FIG. 18).

In addition, it was confirmed that procollagen, a skin aging factor, was expressed similar to or higher than APN5, a conventional known adiponectin-derived peptide, in the group treated with the peptide derivative of the present disclosure (FIG. 19).

### Experimental Example 10. Verification of efficacy in suppressing neutral fat in adipocytes

A mouse adipocyte cell line 3T3-L1 was treated with a differentiation inducing reagent to differentiate into adipocytes, filled with neutral fat, and then treated with the peptide derivatives of the present disclosure at a series of concentrations (10 and 30 µM), cultured for 24 hours, and the cells were obtained and the neutral fat content was measured. As a result, it was confirmed that treatment with the peptide derivative of the present disclosure reduced the neutral fat content that had been made in a concentration-dependent manner (FIG. 20A).

Meanwhile, the mouse adipocyte cell line 3T3-L1 was treated with 10 µM of the peptide derivative of the present disclosure along with a differentiation induction reagent before differentiation induction to differentiate into adipocytes, and then cells were obtained and the neutral fat content was measured. As a result, it was confirmed that treatment with the peptide derivative of the present disclosure inhibited neutral fat accumulation (FIG. 20B).

### Experimental Example 11. Verification of efficacy of alleviating sensitive skin in myocytes

An RD cell line as a human muscle cell line was cultured and then when the cells were 90% full, the medium was replaced with a serum-free medium, and treated with 50 mM lactic acid (LA), a sensitizing skin-causing material, and 10 µM of the peptide derivative of the present disclosure, and after 4 hours, cells were harvested, mRNA was extracted, and the effect on the expression of CGRP as a pain mediator neurotransmitter was observed.

As a result, it was confirmed that when treated with the peptide derivative of the present disclosure, the expression of the pain mediator was significantly reduced compared to when treated with lactic acid, and was at a level similar to or lower than when treated with nothing (FIG. 21). In other words, it may be seen that the peptide derivative of the present disclosure may significantly suppress the expression of genes related to skin sensitivity caused by lactic acid.

As described above, although the examples have been described by the restricted drawings, various modifications and variations may be applied on the basis of the embodiments by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result may be achieved.

Therefore, other implementations, other examples, and equivalents to the appended claims fall within the scope of the claims to be described below.

### INDUSTRIAL APPLICABILITY

The present disclosure relates to a novel peptide derivative, etc., the peptide derivative being a partially modified adiponectin receptor peptide which, compared to conventional adiponectin receptor peptides, has higher stability, superior p-AMPK activity, and improved physical properties and activity and thus offers advantages when formulated into a drug. Therefore, the peptide derivative of the present disclosure may be used to prevent or treat inflammatory skin diseases, wounds, hair loss, fibrosis, metabolic diseases, or cancer, to prevent or alleviate aging, sensitive skin, or wrinkles, or to enhance moisturization.

## Claims

1. A peptide derivative represented by [Chemical Formula 1] below: 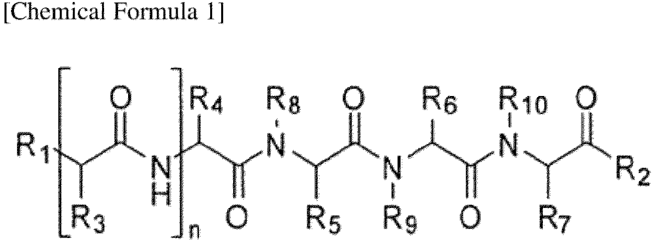
in [Chemical Formula 1],
R₁ is at least one selected from the group consisting of an amino group (NH₂), an acetylamino group (Ac-NH), NH₂-(CH₂CH₂O)ₘ-CH₂CONH, a C₁₅-C₂₀ amide group, and combinations thereof (wherein m is an integer of 1 to 5),
R₂ is an amino group or a hydroxyl group (OH),
R₃ is hydrogen (H),
R₄ is at least one selected from the group consisting of a C₁-C₆ acyclic alkyl group, a C₂-C₆ alkylcarboxyl group, a C₁-C₆ alkylamide group, an imidazoylmethyl group, and combinations thereof,
R₅ and R₆ are the same or different from each other, and are each independently a benzyl group, a phenylethyl group, or an indolylmethyl group, wherein the benzyl group is unsubstituted or at least one selected from the group consisting of a hydroxyl group, a trifluoromethyl group (CF₃), a halogen group, a cyano group, a nitro group, a C₁-C₆ acyclic alkyl group, a C₁-C₆ alkoxy group, and combinations thereof,
R₇ is a benzyl group or a C₁-C₆ alkylamino group, wherein the benzyl group is unsubstituted or at least one selected from the group consisting of a hydroxyl group, a halogen group, a cyano group, a nitro group, a C₁-C₆ acyclic alkyl group, a C₁-C₆ alkoxy group, and combinations thereof,
R₃ and R₇ are linked to each other to form a C₁₅-C₂₀ heterocycloalkene group,
R₈, R₉, and R₁₀ are the same as or different from each other, and are each independently hydrogen or a C₁-C₆ alkyl group, and
n is 0 or 1,
when n is 0, R₄ is a C₂-C₆ alkylcarboxyl group, and R₅, R₆, and R₇ are benzyl groups,
however, R₁ is NH₂, R₂ is OH, R₃ is hydrogen, R₄ is an isobutyl group, R₅ and R₆ are both hydroxybenzyl groups, R₇ is an unsubstituted benzyl group, and R₈, R₉, and R₁₀ are all hydrogen, except for the case where n is 1.

2. The peptide derivative of claim 1, wherein the R₄ is at least one selected from the group consisting of 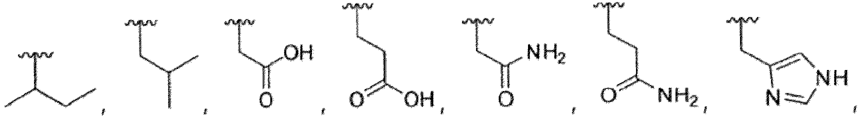 and combinations thereof.

3. The peptide derivative of claim 1, wherein the R₅ and R₆ are the same as or different from each other and each independently at least one selected from the group consisting of 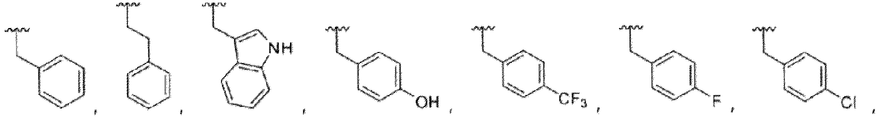 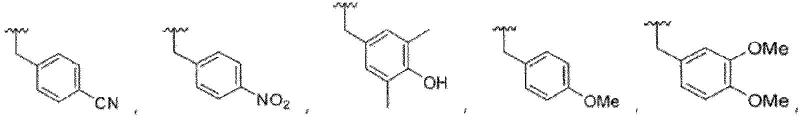 and combinations thereof.

4. The peptide derivative of claim 1, wherein the C₁₅-C₂₀ heterocycloalkene group is at least one selected from the group consisting of 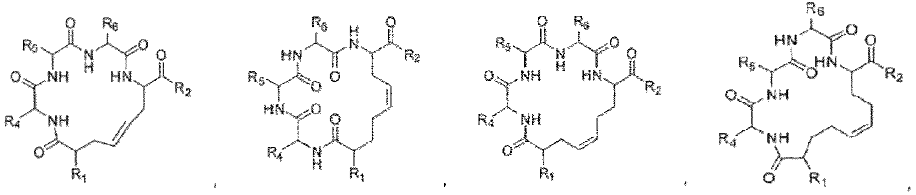 and combinations thereof.

5. The peptide derivative of claim 1, wherein
the peptide derivative is at least one selected from the group consisting of peptide derivatives represented by [Formula 1-1] to [Formula 1-56] below: 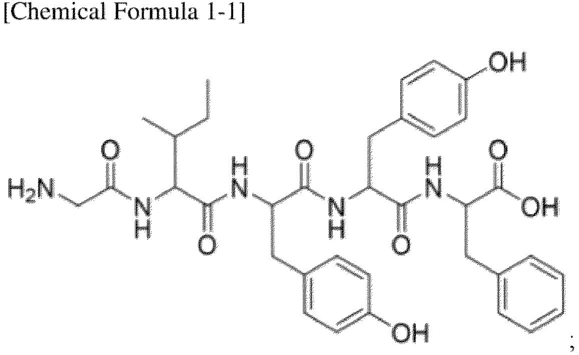 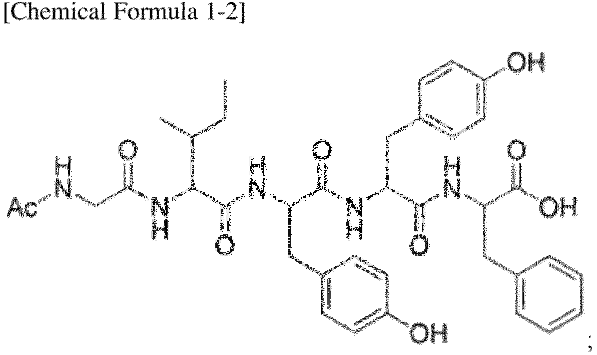 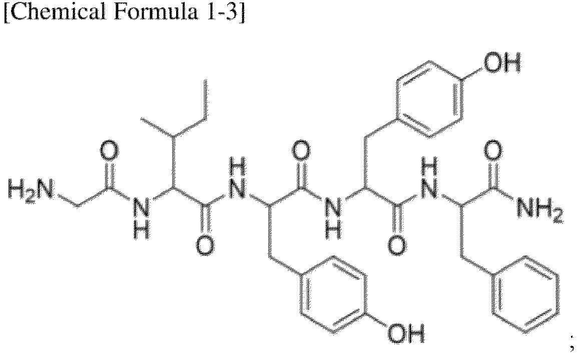 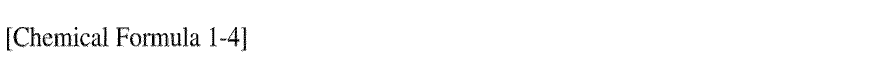 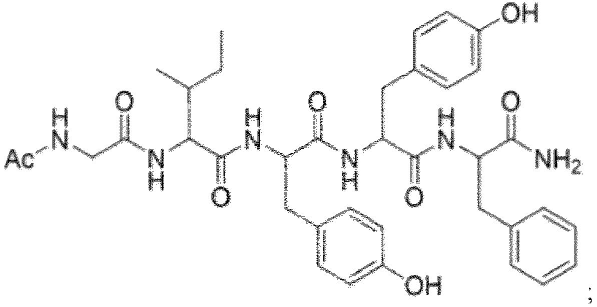 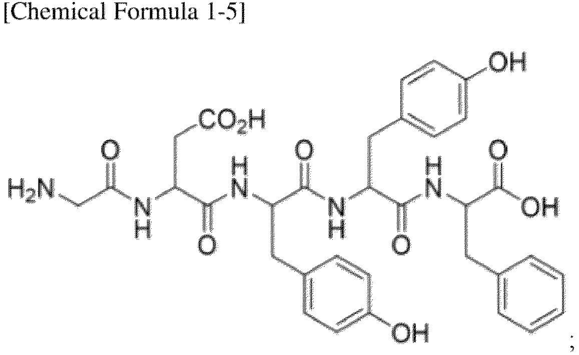 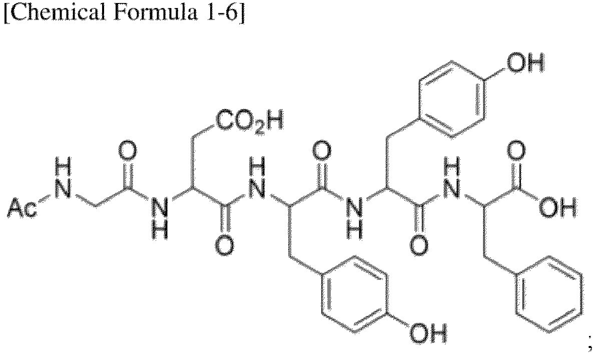 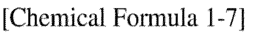 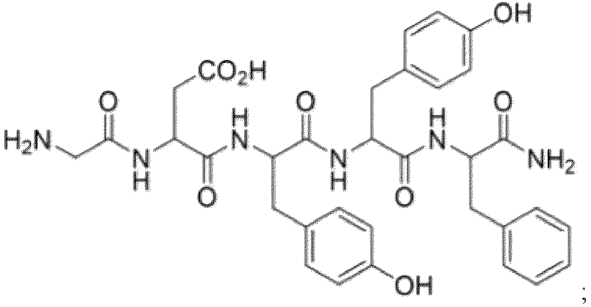 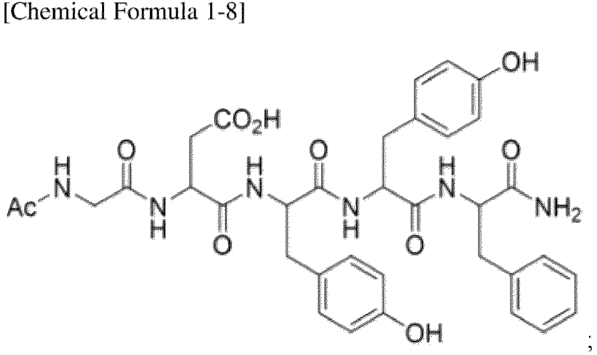 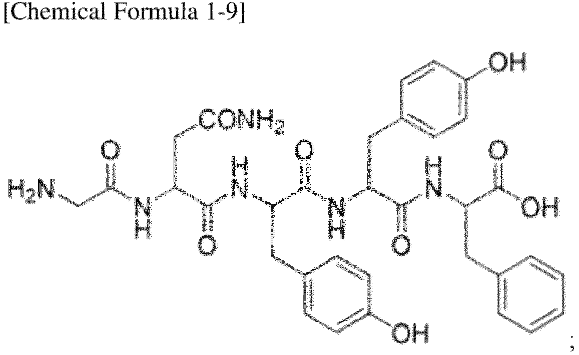 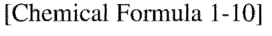 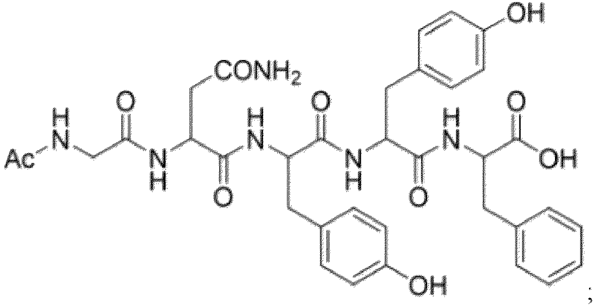 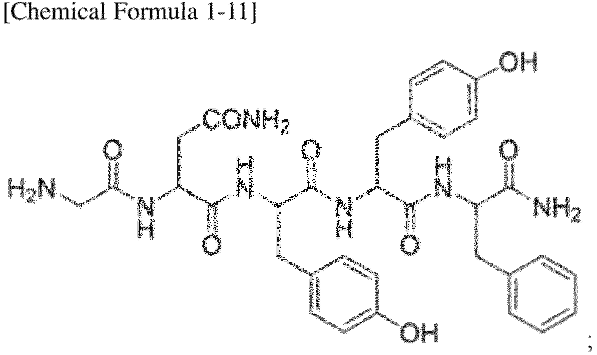 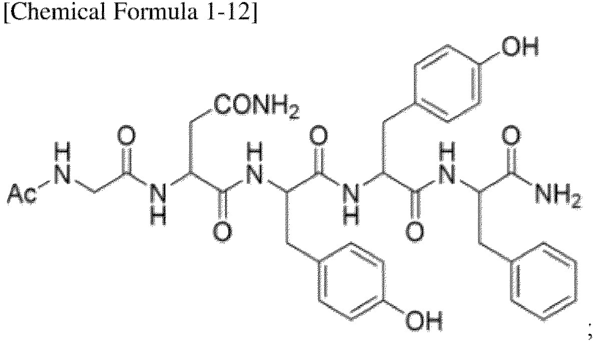 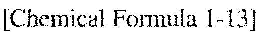 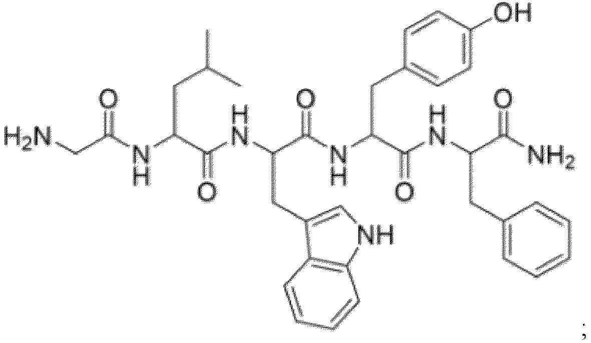 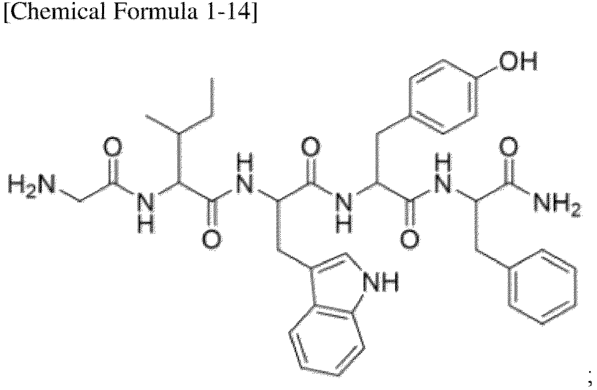 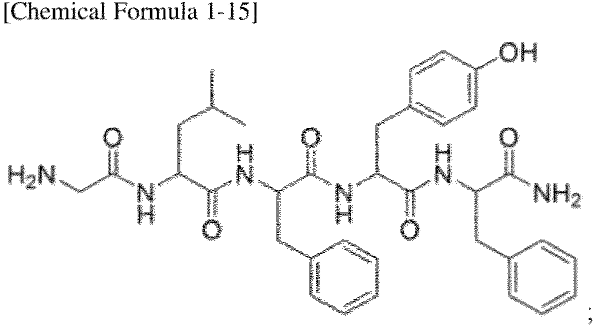 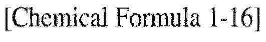 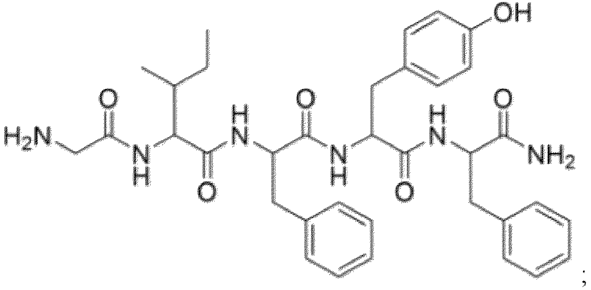 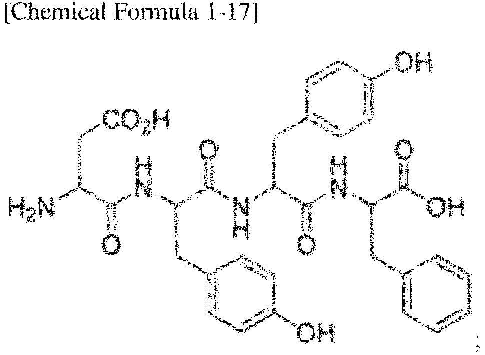 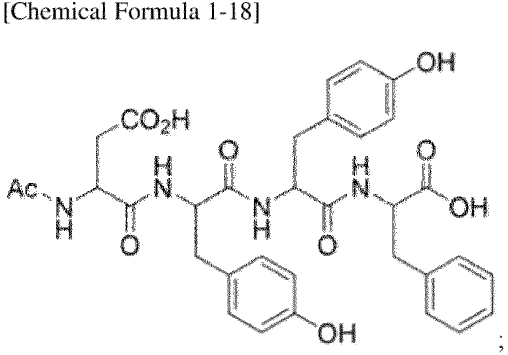 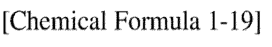 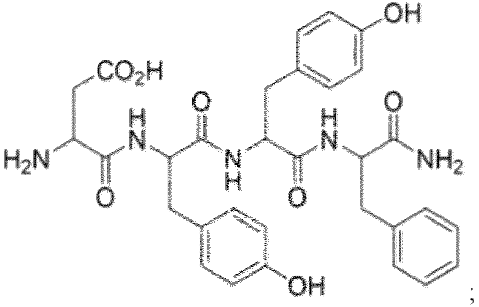 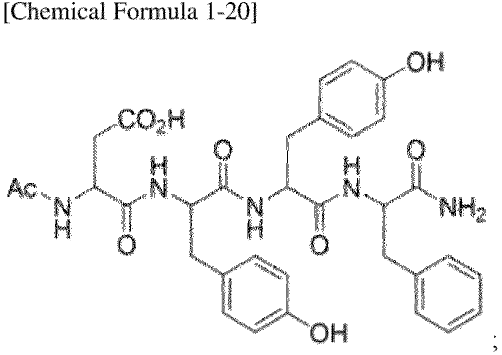 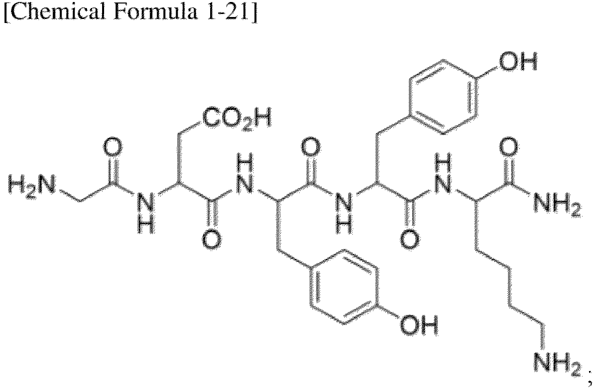 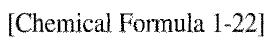 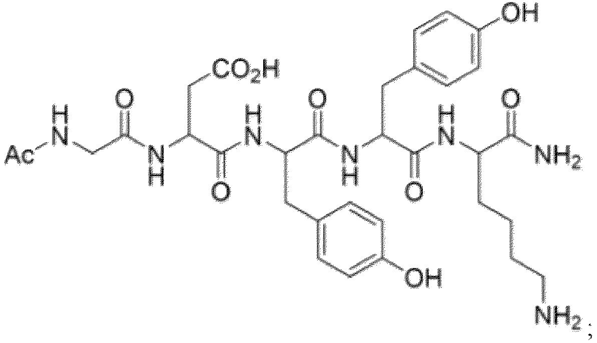 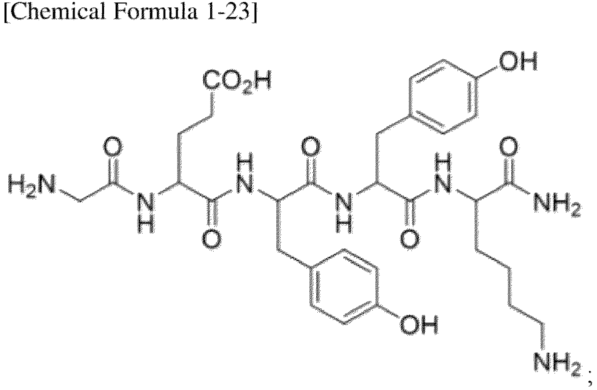 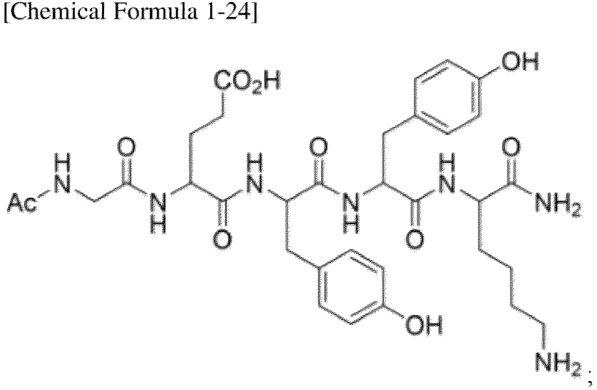 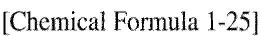 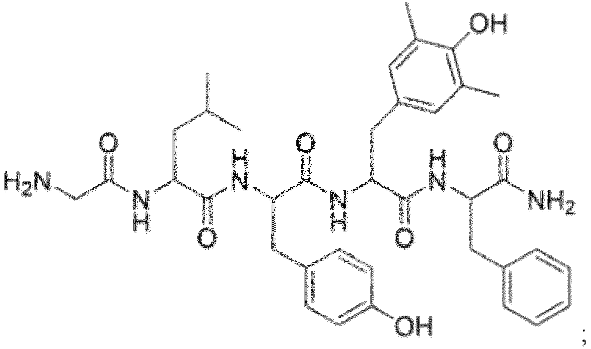 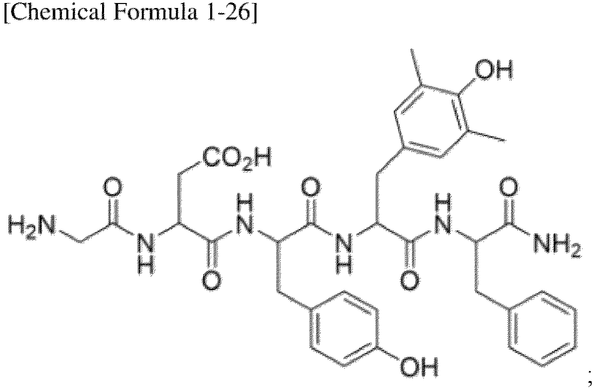 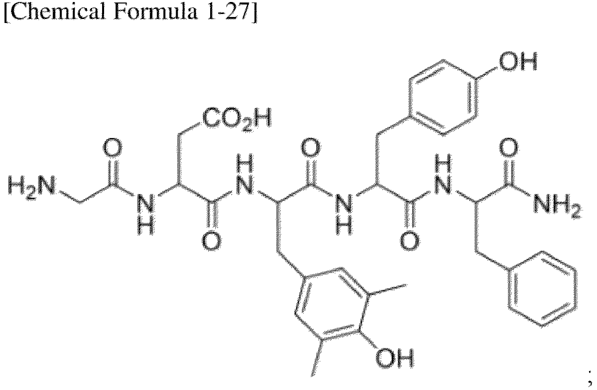 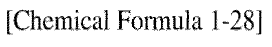 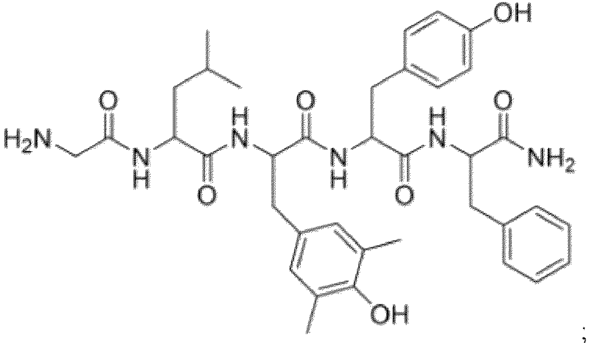 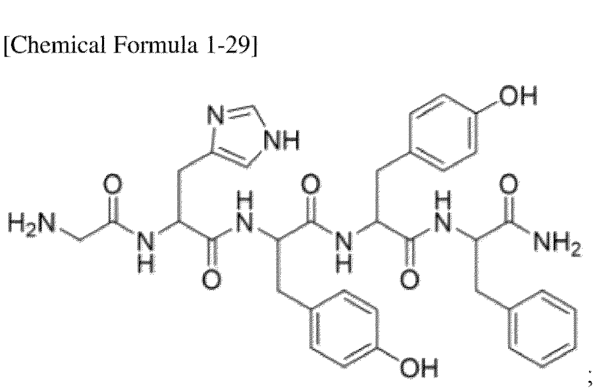 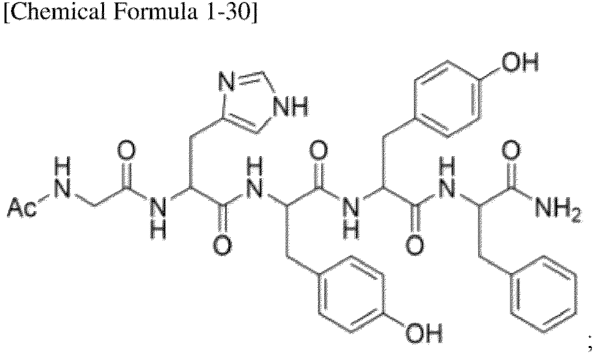 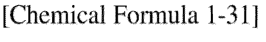 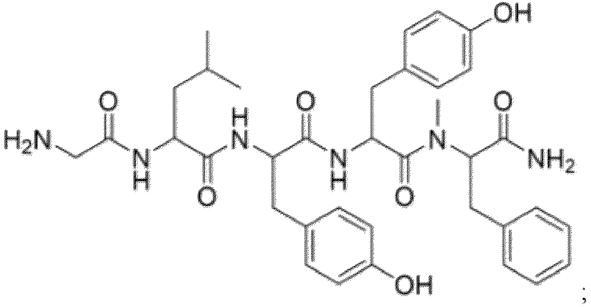 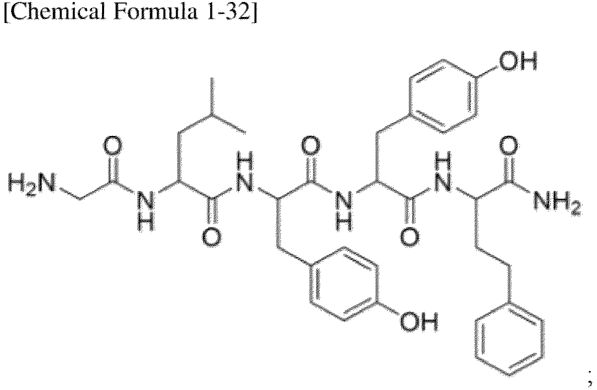 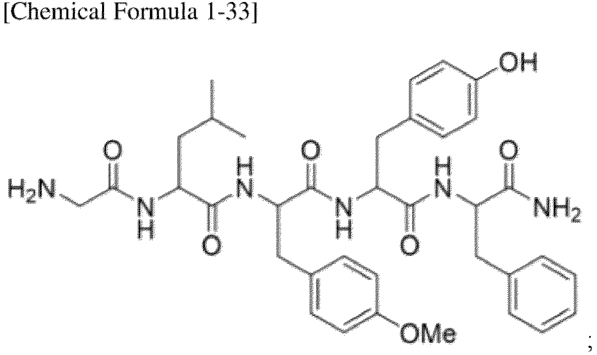 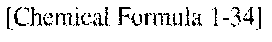 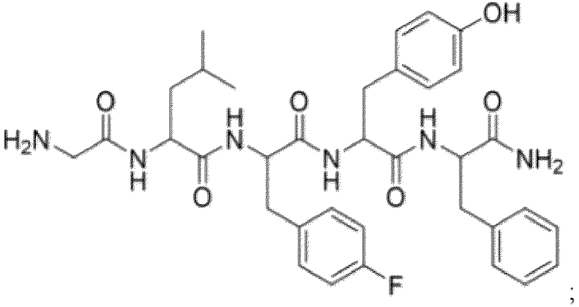 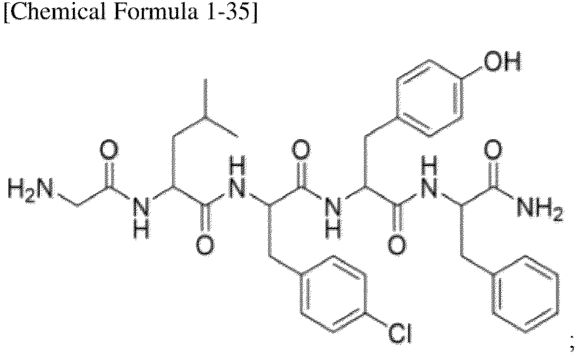 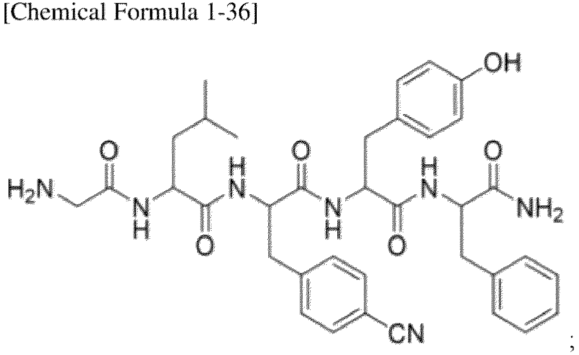 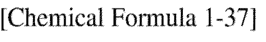 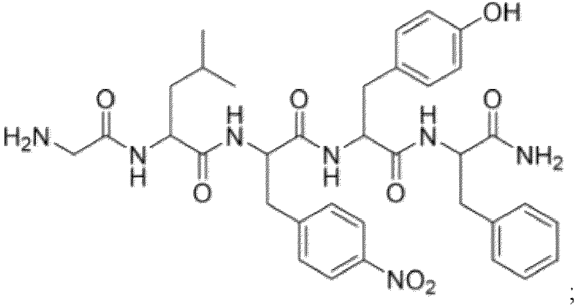 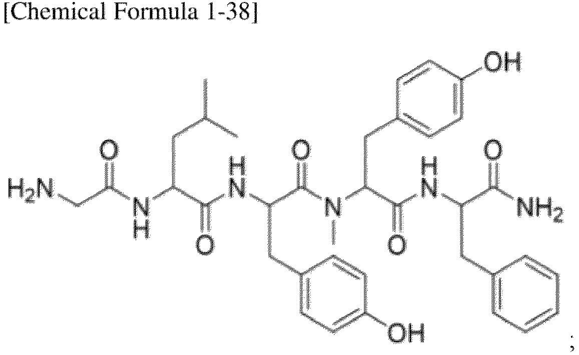 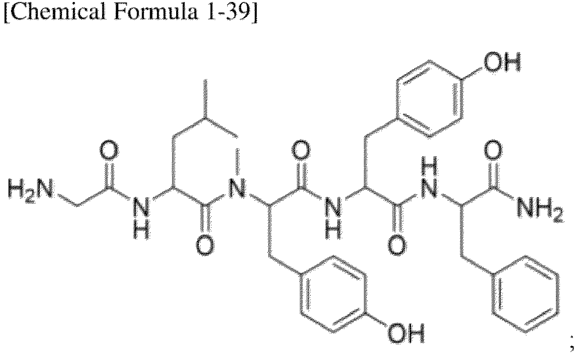 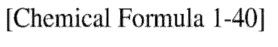 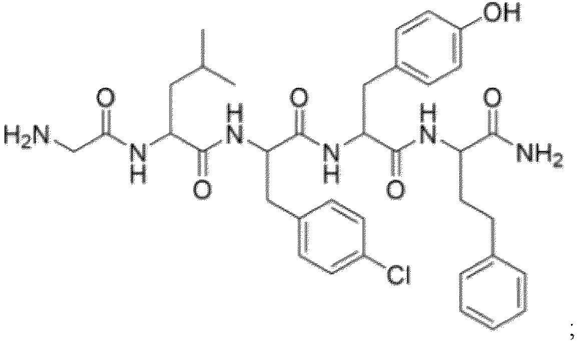 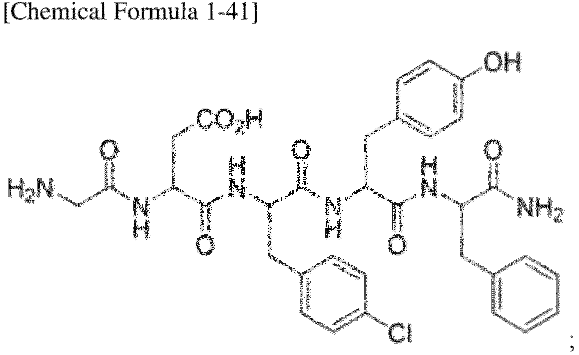 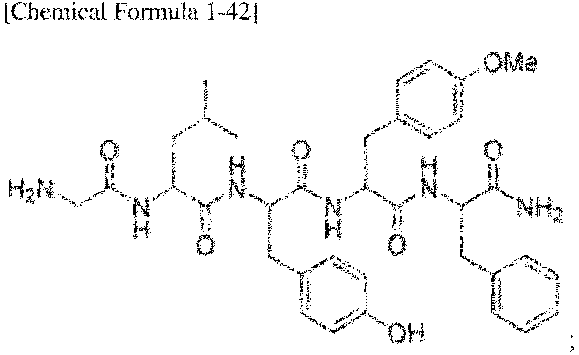 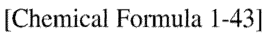 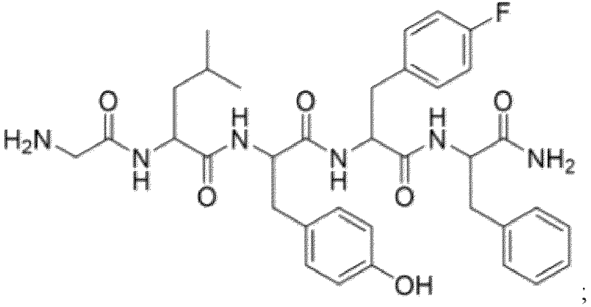 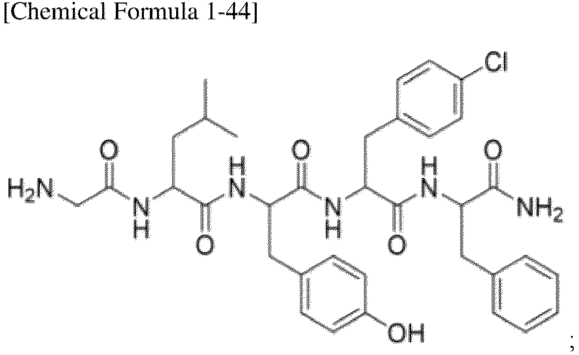 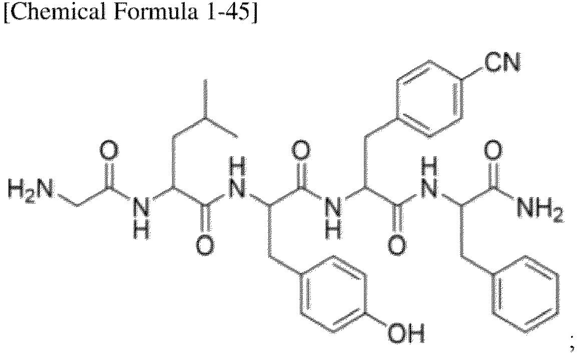 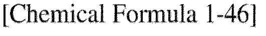 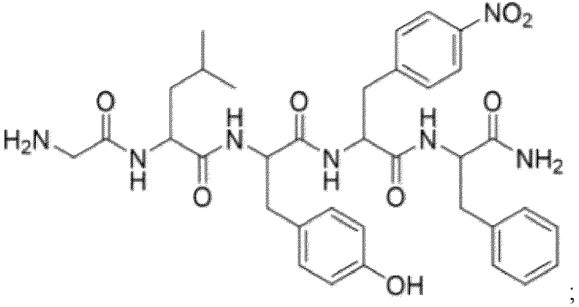 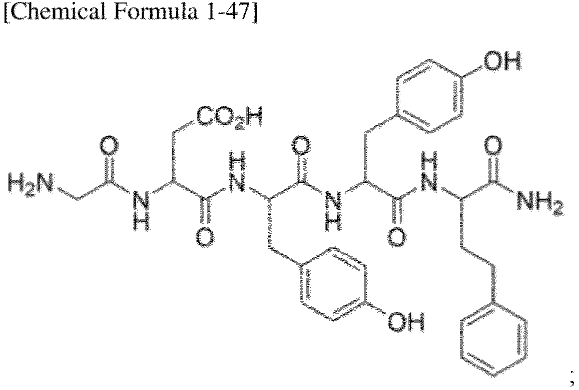 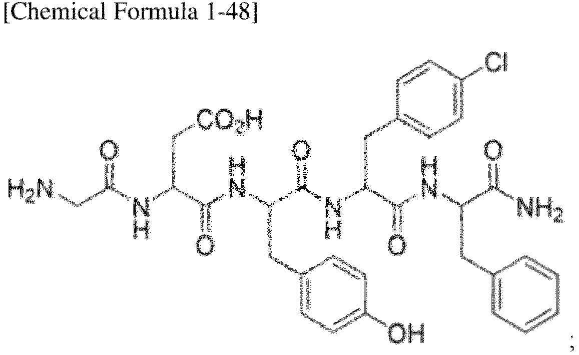 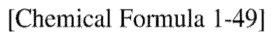 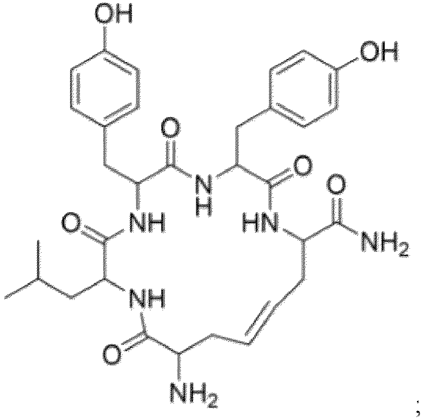 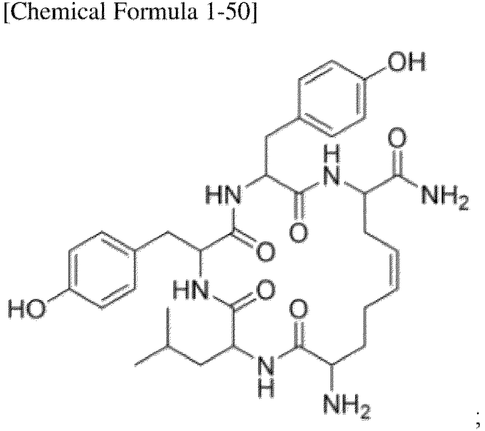 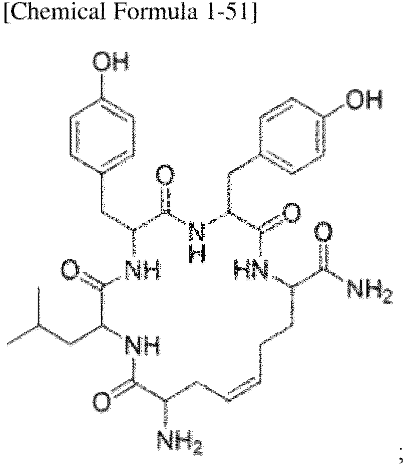 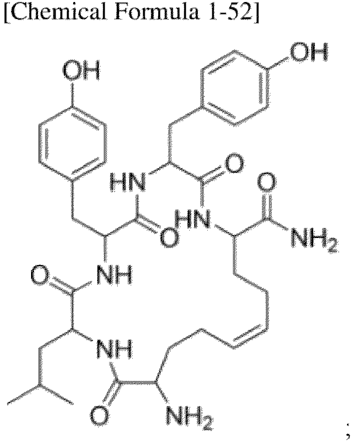 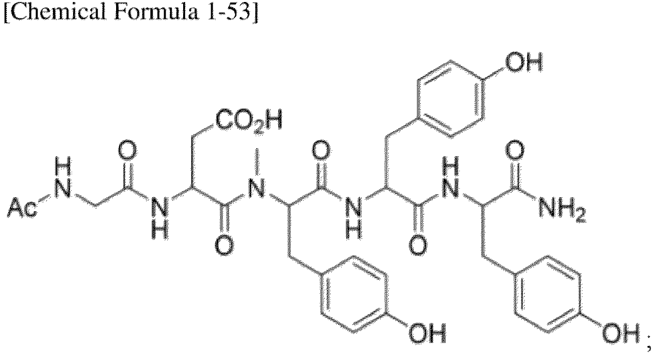 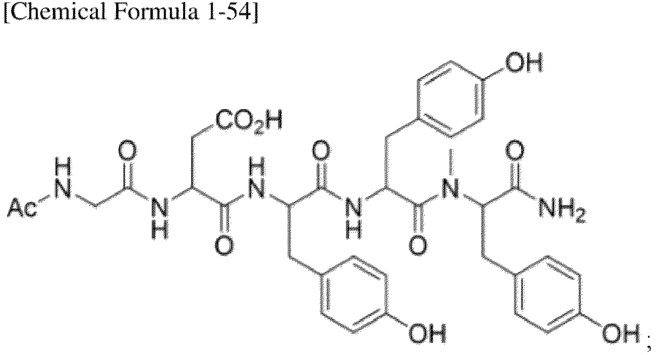 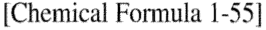 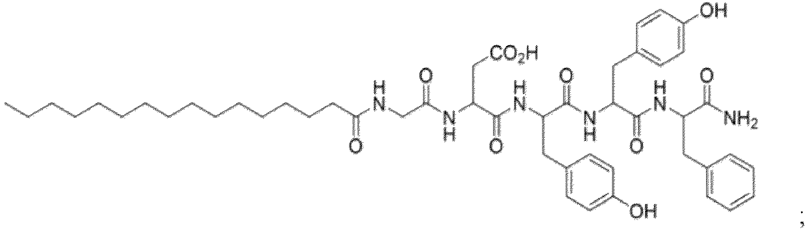 and

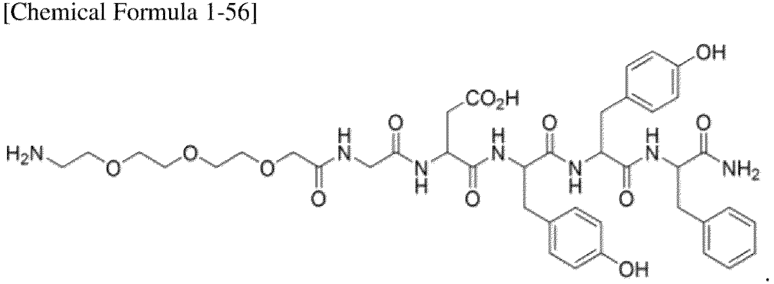

6. The peptide derivative of claim 1, wherein the peptide derivative is at least one modified peptide sequence selected from the group consisting of SEQ ID NOs: 1 to 16.

7. A composition for increasing the expression of adiponectin comprising the peptide derivative of any one of claims 1 to 6 as an active ingredient.

8. A pharmaceutical composition for preventing or treating at least one selected from the group consisting of inflammatory skin diseases, wounds, hair loss, fibrosis, metabolic diseases, and cancer, comprising the peptide derivative of any one of claims 1 to 6 as an active ingredient.

9. A cosmetic composition for preventing or alleviating at least one selected from the group consisting of inflammatory skin diseases, wounds, hair loss, fibrosis, metabolic diseases, and cancer, comprising the peptide derivative of any one of claims 1 to 6 as an active ingredient.

10. A pharmaceutical composition for preventing or alleviating at least one selected from the group consisting of aging, sensitive skin, wrinkles and moisturization, comprising the peptide derivative of any one of claims 1 to 6 as an active ingredient.

11. A cosmetic composition for preventing or alleviating at least one selected from the group consisting of aging, sensitive skin, wrinkles and moisturization, comprising the peptide derivative of any one of claims 1 to 6 as an active ingredient.

12. A method for preventing or treating at least one selected from the group consisting of inflammatory skin diseases, wounds, hair loss, fibrosis, metabolic diseases, and cancer, comprising administering the peptide derivative of any one of claims 1 to 6 to a subject.

13. A method for preventing or alleviating at least one selected from the group consisting of aging, sensitive skin, wrinkles and moisturization, comprising administering the peptide derivative of any one of claims 1 to 6 to a subject.
